# EUROPEAN PATENT APPLICATION

(11) **EP 4 632 067 A1**
(43) Date of publication of application: **15.10.2025**
(21) Application number: 23899101.2
(22) Date of filing: 08.12.2023
(51) Int. Cl.: C12N 15/09, C12N 15/04, C12N 15/05, C12N 15/06

(54) **METHOD FOR PRODUCING GENOME-EDITED CELLS AND METHOD FOR PROMOTING HYBRIDIZATION**

(30) Priority: 08.12.2022 JP 2022196304
(71) Applicant: TOKYO UNIVERSITY OF SCIENCE FOUNDATION, Tokyo 162-8601 (JP)
(72) Inventor: KAMAKURA, Takashi, Tokyo 162-8601 (JP); ARAZOE, Takayuki, Tokyo 162-8601 (JP); KUNIBA, Ryo, Tokyo 162-8601 (JP); CHIBA, Ryo, Tokyo 162-8601 (JP); UCHIDA, Momotaka, Tokyo 162-8601 (JP)
(74) Representative: Isarpatent
(86) International application number: PCT/JP2023/044113
(87) International publication number: WO 2024/122646

(57) **Abstract**

A method of preparing a genome-edited cell, in which a nucleic acid contained in a donor nucleic acid is introduced into the genome of a fused cell during the meiosis process thereof, and a method of promoting hybridization in which two or more types of cells are caused to hybridize during the meiosis process of the fused two or more types of cells having different traits.

## Description

### Technical Field

The present disclosure relates to a method for preparing genome-edited cells and a method for promoting hybridization.

### Background Art

Genome editing technology is used in a wide variety of fields including elucidation of life phenomena, preparation of disease model cells and animals, development of drugs, cell therapy, gene therapy, improvement of agricultural and livestock products, and microbial production of useful substances. As gene editing technology, various techniques that use site-specific nucleases such as zinc finger nuclease (ZFN), TALEN, and CRISPR-Cas9 are known. For example, Patent Literature 1 discloses a method of preparing a mutant filamentous fungus, including introducing a site-specific DNA nuclease and single-stranded DNA into a host filamentous fungus, and substituting an upstream region and a downstream region of a site of the genomic DNA of the host cleaved by the site-specific DNA nuclease with a single strand through homologous recombination.

As a method for acquiring genetic diversity for breed improvement or the like, a method of hybridizing heterologous cells with each other and a method of inducing genomic rearrangement are known. For example, Patent Literatures 2 to 4 disclose a method for promoting genomic rearrangement by allowing a DNA double-strand cleavage enzyme to act on polyploid cells or heteroploid cells prepared by, for example, cell fusion. Patent Literature 5 discloses a method of increasing homologous recombination in dividing cells by introducing into the cells a nucleic acid that encodes a fusion protein containing a DNA binding domain operably linked to the Spo11 protein, and allowing the cells to divide so as to increase homologous recombination. Patent Literature 6 discloses a method for improving a yeast strain, including promoting recombination by using a phenomenon called Return to Growth (RTG), that is, the reversible induction of a yeast to enter meiotic phase I.

### Citation List

### Patent Literature

Patent Literature 1: International Publication (WO) No. 2017/135317
Patent Literature 2: Japanese Patent Application Laid-Open (JP-A) No. 2012-044883
Patent Literature 3: Japanese Patent Application Laid-Open (JP-A) No. 2017-012145
Patent Literature 4: Japanese Patent Application Laid-Open (JP-A) No. 2020-022517
Patent Literature 5: Japanese National Phase Publication (JP-A) No. 2005-532823
Patent Literature 6: Japanese National Phase Publication (JP-A) No. 2015-536663

### SUMMARY OF INVENTION

### Technical Problem

In order to widen the options of genome editing and gene hybridization, the development of novel methods that are different from conventional methods has been desired. In view of such circumstances, the disclosure relates to a novel method for preparing genome-edited cells and a novel method for promoting hybridization.

### Solution to Problem

The disclosure includes the following aspects.
(1) A method of preparing a genome-edited cell, the method including:
   fusing cells; and
   introducing into the cells before, during, or after the fusing: a donor nucleic acid; and a protein that promotes at least one selected from the group consisting of cell fusion, nuclear fusion, and meiosis, or a gene thereof,
   wherein a nucleic acid contained in the donor nucleic acid is introduced into a genome of the cell during meiosis process of the fused cell.
(2) The method of preparing a genome-edited cell according to (1), wherein at least one selected from the group consisting of the fusing of the cells, the introducing of the donor nucleic acid, and the introducing of the protein or the gene thereof is performed by bringing the cells into contact with polyethylene glycol, infecting the cells with a virus, or by using an electrical technique.
(3) The method of preparing a genome-edited cell according to (1) or (2), wherein the cells are plant cells, fungal cells, or animal cells.
(4) The method of preparing a genome-edited cell according to any one of (1) to (3), wherein the cells are filamentous fungal cells.
(5) The method of preparing a genome-edited cell according to any one of (1) to (4), wherein the protein or the gene thereof includes at least one selected from the group consisting of a cell fusion gene, a nuclear fusion gene, a mating-related gene, a meiosis-related gene, and a protein encoded by any of these genes.
(6) The method of preparing a genome-edited cell according to (5), wherein:
   the cell fusion gene includes at least one selected from the group consisting of symc, symb, ham5, ste11, pmk1, whi2, mak1, mak2, and so;
   the nuclear fusion gene includes at least one selected from the group consisting of kar5 and prm1;
   the mating-related gene includes at least one selected from the group consisting of pro 1 and a gene within a MAT idiomorph; and
   the meiosis-related gene includes at least one selected from the group consisting of rad51, ddnm1, CD, CDA, Ung, mre11, rec8, and spo11.
(7) The method of preparing a genome-edited cell according to (6), wherein the gene within a MAT idiomorph includes at least one selected from the group consisting of MAT1-2-1 (MAT hmg1), MAT1-2-2 (MAT hmg2), MAT1-1-1 (MAT α-1), MAT1-1-2 (MAT α-2), and MAT1-1-3 (MAT α-3).
(8) The method of preparing a genome-edited cell according to any one of (1) to (7), wherein introduction of the nucleic acid contained in the donor nucleic acid into the genome of the cell occurs by homologous recombination.
(9) The method of preparing a genome-edited cell according to any one of (1) to (8), further including introducing a nuclease or a gene thereof into the cells before the meiosis.
(10) A method of promoting hybridization, the method including
   fusing two or more types of cells having different traits; and
   introducing a protein that promotes at least one selected from the group consisting of cell fusion, nuclear fusion, and meiosis, or a gene thereof, a nuclease or a gene thereof, or a combination thereof, into the two or more types of cells before, during, or after the fusing,
   wherein the two or more types of cells are caused to hybridize during meiosis of the fused cell.
(11) The method of promoting hybridization according to (10), wherein at least one selected from the group consisting of: the fusing of the cells; and the introducing of the protein or the gene thereof or the nuclease or the gene thereof, is performed by bringing the cells into contact with polyethylene glycol, infecting the cells with a virus, or by using an electrical technique.
(12) The method of promoting hybridization according to (10) or (11), wherein the cells are plant cells, fungal cells, or animal cells.
(13) The method of promoting hybridization according to any one of (10) to (12), wherein the cells are filamentous fungal cells.
(14) The method of promoting hybridization according to any one of (10) to (13), wherein the protein or the gene thereof includes at least one selected from the group consisting of a cell fusion gene, a nuclear fusion gene, a mating-related gene, a meiosis-related gene, and a protein encoded by any of these genes.
(15) The method of promoting hybridization according to (14), wherein:
   the cell fusion gene includes at least one selected from the group consisting of symc, symb, ham5, ste11, pmk1, whi2, mak1, mak2, and so;
   the nuclear fusion gene includes at least one selected from the group consisting of kar5 and prm1;
   the mating-related gene includes at least one selected from the group consisting of pro 1 and a gene within a MAT idiomorph; and
   the meiosis-related gene includes at least one selected from the group consisting of rad51, ddnm1, CD, CDA, Ung, mre11, rec8, and spo11.
(16) The method of promoting hybridization according to (15), wherein the gene within a MAT idiomorph includes at least one selected from the group consisting of hmg1, hmg2, α-1, α-2, and α-3.
(17) The method of promoting hybridization according to any one of (10) to (16), wherein the nuclease or the gene thereof includes at least one selected from the group consisting of a Cas protein, TALEN, ZFN, a restriction enzyme, and a gene of these.

### Advantageous Effects of Invention

According to the disclosure, a novel method for preparing genome-edited cells and a novel method for promoting hybridization are provided.

### BRIEF DESCRIPTION OF DRAWINGS

Fig. 1 shows the outline of the recombinant detection system for *Pyricularia oryzae* used in the Examples.
Fig. 2 shows the outline of the recombinant detection system for *Pyricularia grisea* used in the Examples.
Fig. 3 shows the gene editing scheme used in the Examples for gene editing of *Pyricularia oryzae.*
Fig. 4 shows the gene editing scheme used in the Examples for gene editing of *Aspergillus sojae.*
Fig. 5 shows the results of real-time PCR for confirming the gene expression of MEIOSIN and RAD51 after cell fusion of human cells (HeLa cells).

### DESCRIPTION OF EMBODIMENTS

Hereinafter, embodiments for carrying out the embodiments of the disclosure will be described in detail. However, the embodiments of the disclosure are not limited to the following embodiments. In the following embodiments, the constituents (including element steps) are not essential unless otherwise specified. The same applies to numerical values and ranges thereof, and the numerical values and ranges should not be construed as limiting the embodiments of the disclosure.

In the disclosure, the term "step" includes not only a step independent of other steps but also a step that cannot be clearly distinguished from other steps as long as the purpose of the step is achieved.

In the disclosure, a numerical range indicated using "(from) ... to ..." includes the numerical values described before and after "to" as the minimum value and the maximum value, respectively.

In the numerical ranges described in a stepwise manner in the disclosure, the upper limit value or the lower limit value of a certain numerical range may be replaced with the upper limit value or the lower limit value of another numerical range specified in a stepwise manner. Further, in the numerical ranges described in the disclosure, the upper limit value or the lower limit value of a numerical range may be replaced with a value specified in the Examples.

In the disclosure, a component may include a plurality of substances corresponding to the component. When a plurality of substances corresponding to a component are present in the composition, the content or amount of the component means the total content or amount of the plurality of substances present in the composition, unless otherwise specified.

Even when an element is expressed in the singular form in the disclosure, the presence of a plurality of elements is not excluded as long as there is no technical contradiction, unless otherwise specified.

In the disclosure, the term "nucleic acid" is a term that encompasses all nucleic acids (e.g., DNA, RNA, analogs thereof, and naturally occurring and artificially synthesized ones) and all nucleic acids to which a low-molecular weight compound, a group, a molecule other than a nucleic acid, or a structure, for example, is linked. The nucleic acid may be a single-stranded nucleic acid or a double-stranded nucleic acid.

In the disclosure, the term "meiosis" refers to a mode of cell division during which the number of chromosomes is reduced by half through pairing and segregation of homologous chromosomes, and does not necessarily mean meiosis that takes place in germ cells.

### <<Method of Preparing Genome-Edited Cell>>

A method of preparing a genome-edited cell according to an embodiment of the disclosure includes:
fusing cells (hereinafter, also referred to as a "cell fusion step"); and
introducing into the cells before, during, or after the fusing: a donor nucleic acid; and a protein that promotes at least one selected from the group consisting of cell fusion, nuclear fusion, and meiosis, or a gene thereof (hereinafter, also referred to as a "donor nucleic acid/promoting factor introduction step"),
wherein a nucleic acid contained in the donor nucleic acid is introduced into the genome of the cell during meiosis of the fused cell. Hereinafter, the "protein that promotes at least one selected from the group consisting of cell fusion, nuclear fusion, and meiosis, or a gene thereof" is also referred to as a "promoting factor".

The inventors have found that genome editing of cells can be achieved by combining cell fusion and the introduction of a promoting factor. The mechanism thereof is presumed to be as follows. Cell fusion and subsequent nuclear fusion result in a polyploid having an increased number of chromosomes. It is assumed that the obtained fused cell has or has acquired the ability to undergo meiosis. Here, the introduction of a donor nucleic acid and a promoting factor promotes meiosis through promotion of cell fusion or nuclear fusion, or by a promoting factor that promotes meiosis. It is presumed that the nucleic acid contained in the donor nucleic acid is introduced into the genome through homologous recombination associated with meiosis. However, it is noted that the embodiments of the disclosure are not limited by the estimated mechanisms.

These findings are supported by, for example, the following discoveries. It has been known that, in filamentous fungi, genetic diversity is achieved by somatic cell fusion without involving sexual organs, although the molecular mechanism thereof has not been elucidated. The inventors have found that pseudo-sexual reproduction that involves hyphal fusion, nuclear fusion, and a meiosis-like reaction is involved in this process.

The inventors found that the frequency of the pseudo-sexual reproduction decreases when a hyphal fusion-related gene ham5 or ste11 is deleted in filamentous fungi. This suggests that the pseudo-sexual reproduction occurs via hyphal fusion. Further, it was found that the frequency of the pseudo-sexual reproduction decreases when other genes that are involved in cell fusion, nuclear fusion, and meiosis are deleted.

It was also found that, when protoplasts of filamentous fungal cells are forcibly fused, nuclear fusion and subsequent pseudo-sexual reproduction, in other words, a meiosis-like reaction, occur, resulting in genomic hybridization.

In addition, it was found that the meiosis-like reaction is promoted by cell fusion also in human cells in the same manner as in filamentous fungi.

These findings suggest that supplementation of various factors that promote cell fusion, nuclear fusion, and/or meiosis can promote meiosis during pseudo-sexual reproduction, and promote the process of homologous recombination. The inventors have conceived of applying the foregoing process to genome editing. According to the study by the inventors, it was found that, while the frequency of homologous recombination is low with cell fusion only, the frequency of homologous recombination is increased when various factors that promote cell fusion, nuclear fusion, and/or meiosis are supplemented at the time of cell fusion. It is believed that this technique is applicable to various cells irrespective of the cell type, and in particular, is applicable to non-germ cells.

The donor nucleic acid/promoting factor introduction step may be performed before, after, or during the cell fusion step.

### <Cell Fusion Step>

In the cell fusion step, multiple cells are fused. The number of cells to be fused may be two or more, for example, two. The method of cell fusion is not limited, and various known methods can be used. For example, cell fusion can be performed by bringing the cells into contact with polyethylene glycol (PEG), infecting the cells with a virus, or using an electrical technique. When cells having cell walls are used, the cell walls are typically removed to obtain protoplasts, and then the cells are fused.

Examples of the cells include eukaryotic cells, such as plant cells, fungal cells, or animal cells.

Examples of the plant cells include cells of rice, wheat, tobacco, tomato, or potato.

Examples of the animal cells include cells of a mammal, such as a human, monkey, mouse, rat, dog, cat, or rabbit; a bird, such as a chicken; an amphibian; a reptile; fish; a chordate; an arthropod; or an insect. The animal cells may be non-human cells and may be non-human embryonic cells (i.e., cells other than human embryonic cells).

Examples of the fungal cells include cells of a filamentous fungus or yeast.

Examples of the filamentous fungus include a filamentous fungus belonging to the genus *Pyricularia, Aspergillus, Penicillium, Trichoderma, Fusarium, Humicola, Talaromyces,* or *Acremonium.* In particular, *Pyricularia oryzae* (also referred to as *Magnaporthe oryzae)* has been well-studied as a rice blast fungus, and *Pyricularia grisea* has been well-studied as a crabgrass blast fungus. *Aspergillus oryzae* and *Aspergillus sojae* are well known as koji molds.

Examples of the yeast include a yeast belonging to the genus *Saccharomyces, Schizosaccharomyces, Pichia, Candida, Kluyveromyces,* or *Moniliella.*

The cells may be non-germ cells (e.g., somatic cells) or germ cells. In particular, the technology disclosed herein is also applicable to non-germ cells, which normally do not undergo meiosis. As the cells, tissue cells or cells derived from tissues, or cells having various properties can be used.

The cells to be fused may be homologous cells having the same trait, or heterologous cells having different traits.

### <Donor Nucleic Acid/Promoting Factor Introduction Step>

In the donor nucleic acid/promoting factor introduction step, a donor nucleic acid and a promoting factor are introduced into the cells before, during, or after the fusion. The donor nucleic acid and the promoting factor may be introduced simultaneously, or either one of them may be introduced first. One type of, or two or more types of, each of the donor nucleic acid and the promoting factor may be introduced. The timing and order of the introduction of the donor nucleic acids and the promoting factor are not particularly limited, as long as they have been introduced by the time of meiosis, and as long as the objective of the disclosure is not impaired.

### -Donor Nucleic Acid-

The donor nucleic acid is a foreign nucleic acid containing a nucleic acid to be introduced into the genome of the cell(s) (hereinafter, also referred to as "nucleic acid insert"). The donor nucleic acid may be a donor nucleic acid used for knock-in, or one used for knockout.

The donor nucleic acid may be a nucleic acid insert linked to a freely-selected vector. Examples of the vector include: a plasmid vector; a viral vector, such as a retrovirus vector, a lentivirus vector, an adenovirus vector, an adeno-associated virus vector, a herpesvirus vector, or a Sendai virus vector; and an *Agrobacterium* vector.

The vector may contain any of various nucleic acid sequences, including: a regulatory sequence, such as a promoter, an enhancer, or a polyadenylation signal; a restriction enzyme cleavage site; a replication origin; a drug resistance gene; or a nucleic acid sequence encoding a marker, such as a fluorescent protein.

Examples of the promoter include a TEF promoter, a TRPC promoter, a CMV promoter, an EF1 promoter, an SV40 promoter, an MSCV promoter, a hTERT promoter, a β-actin promoter, a CAG promoter, a mouse U6-snRNA promoter, a human U6-snRNA promoter, a human H1-RNase P RNA promoter, a human valine-tRNA promoter, and a GPD promoter.

Examples of the restriction enzyme cleavage site include a cleavage site of any of various restriction enzymes, such as AccI, BamHI, EcoRI, HincII, HindIII, I-SceI, KpnI, PstI, SacI, SalI, SmaI, SphI, Sse8387I, TaqI, XbaI, Esp3I, BsaI, SpeI, AscI, and PacI. The vector may contain a multiple cloning site that includes multiple restriction enzyme cleavage sites.

Examples of the drug resistance gene include a chloramphenicol resistance gene, a tetracycline resistance gene, a neomycin resistance gene, an erythromycin resistance gene, a spectinomycin resistance gene, a kanamycin resistance gene, a hygromycin resistance gene, a puromycin resistance gene, a bialaphos resistance gene, a blasticidin S resistance gene, a zeocin resistance gene, and a pyrithiamine resistance gene.

The vector may be a commercially available vector, or may be prepared. The vector can be prepared in accordance with known genetic engineering techniques, such as PCR, restriction enzyme cleavage, DNA ligation techniques, and in vitro transcription/translation techniques.

In an embodiment, the donor nucleic acid may contain homology arms for targeted introduction of the nucleic acid. The homology arms are composed of a combination of a 5' homology arm having a sequence homologous to a nucleic acid sequence upstream (5' side) of the target site (that is, a site to which the nucleic acid is intended to be introduced) on the genome, and a 3' homology arm having a sequence homologous to a nucleic acid sequence downstream (3' side) of the target site on the genome. The number of bases in each of the homology arms on the 5' side and the 3' side may be, for example, from 5 to 10,000, or from 100 to 1000. When the donor nucleic acid contains homology arms, the nucleic acid insert is disposed between the two homology arms.

In a case in which the donor nucleic acid itself has sufficient homology with the target site of the genome, homologous recombination with a sequence on the genome can occur even without homology arms. Therefore, for modification of the sequence, the donor nucleic acid may be a nucleic acid that differs from the target site by several bases, or a nucleic acid that has an insertion or deletion. In this case, the combination of the 5' homology arm having a sequence homologous to the nucleic acid sequence upstream (5' side) of the target site and the 3' homology arm having a sequence homologous to the nucleic acid sequence downstream (3' side) of the target site on the genome does not necessarily need to be used, and a nucleic acid having a difference from the target sequence may be used. The number of bases of the donor nucleic acid that are identical to the target site may be, for example, from 5 to 10,000, or from 100 to 1000.

The donor nucleic acid can be introduced into the cell(s) in accordance with a method known in the art depending on the type of cell(s). Examples thereof include a method of bringing the cell(s) into contact with polyethylene glycol (typically the protoplast-PEG method), a method of infecting the cell(s) with a virus, and an electrical technique (typically electroporation).

In particular, for animal cell(s), bringing the cell(s) into contact with polyethylene glycol, viral infection, an electrical technique, or the like can be easily carried out.

For plant cell(s), bringing the cell(s) into contact with polyethylene glycol, an electrical technique, or the like can be easily carried out.

For fungal cell(s), the protoplast-PEG method, an electrical technique, or the like can be easily carried out.

### -Promoting Factor-

The promoting factor is not limited as long as it is a protein that promotes at least one selected from the group consisting of cell fusion, nuclear fusion, and meiosis, or a gene thereof. The promoting factor preferably includes at least one selected from the group consisting of a cell fusion gene, a nuclear fusion gene, a mating-related gene, a meiosis-related gene, and a protein encoded by any of these genes.

Examples of the cell fusion gene include symc, symb, ham5, ste11, pmk1, whi2, mak1, mak2, and so.

Examples of the nuclear fusion gene include kar5 and prm1.

Examples of the mating-related gene include pro1 and a gene within a MAT idiomorph. Examples of the gene within a MAT idiomorph include MAT1-2-1 (MAT hmg1), MAT1-2-2 (MAT hmg2), MAT1-1-1 (MAT α-1), MAT1-1-2 (MAT α-2), and MAT1-1-3 (MAT α-3).

Examples of the meiosis-related gene include rad51, ddnm1, CD, CDA, Ung, mre11, rec8, and spo11.

The promoting factor is not limited to the above examples. The promoting factor can be identified by deleting a gene in a cell and observing the presence or absence of, or frequency of occurrence of, at least one selected from the group consisting of cell fusion, nuclear fusion, and meiosis. Specifically, when the occurrence of the foregoing event disappears, or the frequency of occurrence thereof decreases, due to the deletion of a gene, the gene or a corresponding protein can be identified as a promoting factor. The promoting factor can be obtained by obtaining the gene sequence or amino acid sequence of the identified promoting factor.

When the promoting factor is a protein, the method for introducing the promoting factor into the cell(s) is not particularly limited. Examples include contacting the cell(s) with polyethylene glycol, and an electrical technique.

When the promoting factor is a gene, the method for introducing the promoting factor into the cell(s) is not particularly limited. For example, any of the vectors specifically described in the section on the "Donor Nucleic Acid" can be introduced into the cell(s) by a method known in the art. The vector for the donor nucleic acid and the vector for introducing a promoting factor may be the same or different. From the viewpoint of easily providing homology (homology arms), different vectors are preferably used.

In the method of preparing a genome-edited cell according to the disclosure, a nucleic acid (nucleic acid insert) contained in the donor nucleic acid is introduced into the genome of the cell during the meiosis of the fused cell. The nucleic acid insert can be introduced into the genome by homologous recombination. In an embodiment, for example, when the cells prior to the fusion are haploid cells, a diploid cell is generated by cell fusion and nuclear fusion, and thereafter haploid cells are generated by meiosis. In the haploid cells thus obtained, the nucleic acid insert can be introduced as a result of homologous recombination.

Prior to meiosis, a nuclease or a gene thereof may be further introduced into the cell(s). In the method of preparing a genome-edited cell according to the disclosure, genome editing can occur along with meiosis even if a nuclease is not added. This is believed to be due to, for example, cleavage of a double strand or a single strand, or induction of nucleic acid excision repair, by cellular endogenous nucleases or modification enzymes (e.g., Spo11, CDA, Ung), and subsequent homologous recombination. In contrast, when an exogenous nuclease is introduced, it is believed that homologous recombination is further promoted, resulting in improved genome editing efficiency.

As the nuclease, a double-strand cleaving nuclease is preferable. Examples of the nuclease include a Cas protein (e.g., Cas9), TALEN, ZFN, and a restriction enzyme. Examples of the restriction enzyme include restriction enzymes exemplified in the section on the "Donor Nucleic Acid".

Examples of the method for introducing a nuclease or a gene thereof into the cell(s) include the methods exemplified as the method for introducing a promoting factor into the cell(s). The introduction of a nuclease or a gene thereof into the cell(s) may be performed at any time before meiosis, for example, before or after the cell fusion, before or after the introduction of a donor nucleic acid, or before or after the introduction of a promoting factor.

In an embodiment, at least one selected from the group consisting of the cell fusion, the introduction of a donor nucleic acid, and the introduction of a promoting factor may be performed by bringing the cell(s) into contact with polyethylene glycol, infecting the cell(s) with a virus, or using an electrical technique.

The cell fusion, introduction of a donor nucleic acid, and introduction of a promoting factor may be performed by the same or different methods. The cell fusion, introduction of a donor nucleic acid, and introduction of a promoting factor may be performed successively or sequentially. For example, cell fusion, introduction of a donor nucleic acid, and introduction of a promoting factor can be performed in a sequence of operations by bringing the cells into contact with polyethylene glycol.

### <<Method of Promoting Hybridization>>

A method of promoting hybridization according to an embodiment of the disclosure includes:
fusing two or more types of cells having different traits (hereinafter, also referred to as a "cell fusion step"); and
introducing a protein that promotes at least one selected from the group consisting of cell fusion, nuclear fusion, and meiosis, or a gene thereof, a nuclease or a gene thereof, or a combination thereof, into the two or more types of cells before, during, or after the fusing (hereinafter, also referred to as a "promoting factor/nuclease introduction step"),
wherein the two or more types of cells are caused to hybridize during meiosis of the fused cell. Also in this method, "a protein that promotes at least one selected from the group consisting of cell fusion, nuclear fusion, and meiosis, or a gene thereof" is referred to as a "promoting factor".

The inventors found that hybridization between heterologous cells can be promoted by combining cell fusion and the introduction of a promoting factor and/or a nuclease. The mechanism thereof is presumed to be as follows. Cell fusion causes formation of a polyploid having an increased number of chromosomes. It is assumed that the obtained fused cell potentially has or has acquired the ability to undergo meiosis. Here, the introduction of a promoting factor and/or a nuclease promotes homologous recombination during meiosis, through the promotion of cell fusion or nuclear fusion, through a promoting factor that promotes meiosis, or through cleavage of the genome by a nuclease. As a result, it is believed that hybridization between heterologous cells is promoted. These findings are also supported by the discoveries in the aforementioned filamentous fungi and human cells. However, it is noted that the embodiments of the disclosure are not limited by the estimated mechanism.

### <Cell Fusion Step>

The cell fusion step in the method of promoting hybridization can be performed by the same methods as those applied in the cell fusion step in the method of preparing a genome-edited cell described above.

However, the cells to be fused are two or more types of cells having different traits. By fusing two or more types of cells having different traits, hybridization of genomes having different genetic information through homologous recombination can be promoted. The term "trait" means the genetic phenotype or nature of a cell. It is noted that a feature that is observable as an external feature is expressed as a "phenotype", whereas a feature that is not observable as an external feature is expressed as a "nature". A trait may be inherited in a dominant manner, in a recessive manner, or in a partially or incompletely dominant manner. The trait may be monogenic or polygenic.

Examples of the trait include color, shape, disease resistance, moisture resistance, heat resistance, drug resistance, yield, fruiting property, growth rate, substance productivity, cold resistance, genome stability, and genome instability.

It is noted that the "two or more cells having different traits" may be cells that belong to the same biological classification (e.g., genus, species), or may be cells that belong to different biological classifications, as long as they are cells having mutually different traits.

The method of promoting hybridization according to the disclosure enables even cells that normally cannot be hybridized with each other to be hybridized through cell fusion followed by meiosis.

### <Promoting Factor/Nuclease Introduction Step>

In the promoting factor/nuclease introduction step, a promoting factor, a nuclease or a gene thereof, or a combination thereof, is introduced into two or more types of cells before, during, or after the fusion. One type of, or two or more types of promoting factor or a nuclease or a gene thereof may be introduced. The timing and order of introducing the promoting factor or a nuclease or a gene thereof are not particularly limited, as long as they have been introduced by the time of meiosis, and as long as the objective of the disclosure is not impaired. When a promoting factor and a nuclease or a gene thereof are introduced in combination, the timing and order of introducing them are not particularly limited.

### -Promoting Factor-

Details of the promoting factor are the same as those described in the section on the "Method of Preparing Genome-Edited Cell".

### -Nuclease or Gene Thereof-

As the nuclease, a nuclease involved in double-strand cleavage, single-strand cleavage, or nucleic acid excision repair, or a modification enzyme is preferable. Examples of the nuclease include a Cas protein (e.g., Cas9), TALEN, and a restriction enzyme. Examples of the restriction enzyme include the restriction enzymes exemplified in the section on the "Donor Nucleic Acid". Examples of the modification enzyme include CD, CDA, Ung, or a combination thereof.

Examples of the method for introducing a promoting factor, a nuclease or a gene thereof, or a combination thereof into the cell(s) include the methods specifically described as the method for introducing a promoting factor into the cell(s).

In an embodiment, at least one selected from the group consisting of fusion of cells and introduction of a promoting factor or a nuclease or a gene thereof may be performed by bringing the cell(s) into contact with polyethylene glycol, infecting the cell(s) with a virus, or using an electrical technique.

The fusion of cells and the introduction of a promoting factor, a nuclease, or a gene thereof may be performed by the same or different methods. The fusion of cells and introduction of a promoting factor or a nuclease or a gene thereof may be performed in a sequence of operations, or intermittently. For example, the fusion of cells and the introduction of a promoting factor or a nuclease or a gene thereof can be performed in a sequence of operations by bringing the cells into contact with polyethylene glycol.

### [Examples]

Next, embodiments of the disclosure will be specifically described by way of examples. However, the embodiments of the disclosure are not limited to these examples.

### 1. Elucidation of Pseudo-Sexual Reproduction Mechanism by Gene Defects

It has been known that, in filamentous fungi, genetic diversity is achieved by somatic cell fusion without involving sexual organs, although the molecular mechanism thereof has not been elucidated. The inventors have found that somatic homologous recombination occurs via hyphal fusion in filamentous fungi, *Pyricularia oryzae* and *Pyricularia grisea.* In order to elucidate the mechanism of this phenomenon, the inventors examined the roles of various genes by deleting the genes.

### 1-1. Pseudo-Sexual Reproduction in Pyricularia oryzae

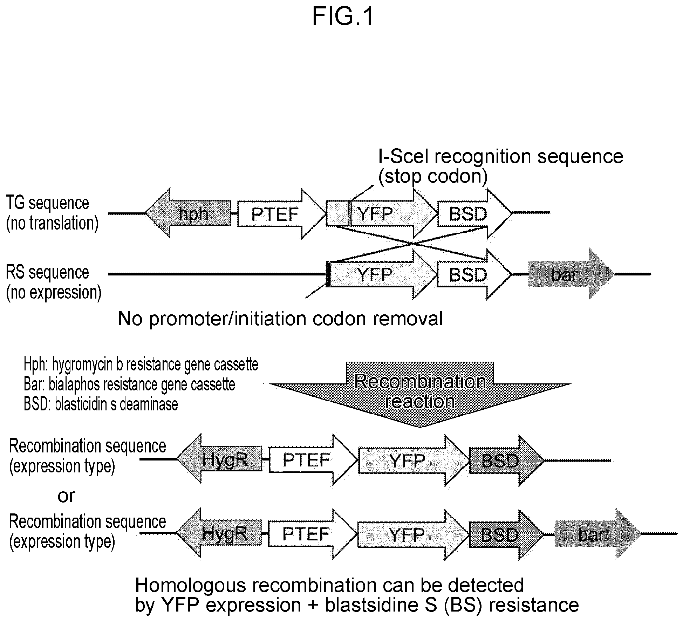

In the following experiment, as an experimental system for detecting recombination, a *Pyricularia oryzae* strain having the following TG sequence introduced therein (TG strain) and a *Pyricularia oryzae* strain having the RS sequence introduced therein (RS strain) were used. The outline of the recombination detection system is shown in Fig. 1.

The TG sequence has, in the order from the 5' end, a hygromycin B resistance gene cassette (HygR), a TEF promoter (PTEF), a yellow fluorescent protein (YFP) gene, and a blasticidin S deaminase (BSD) gene. An I-SceI recognition sequence is inserted within the YFP gene, and a stop codon is inserted into the recognition sequence. YFP and BSD are not translated in a cell having the TG sequence alone.

The RS sequence has, in the order from the 5' end, a yellow fluorescent protein (YFP) gene, a blasticidin S deaminase (BSD) gene, and a bialaphos resistance gene cassette (BlpR). Since the promoter and the initiation codon of the YFP gene are deleted, YFP and BSD are not expressed in a cell having an RS sequence alone.

In contrast, when homologous recombination occurs between the TG sequence and the RS sequence through chromosomal crossover, the host cell acquires blasticidin S resistance, and YFP fluorescence is generated. At this time, when chromosome exchange occurs due to the crossover reaction, triple resistance, namely, hygromycin B resistance, blasticidin S resistance, and bialaphos resistance, is acquired, and YFP fluorescence is generated. This phenomenon was used to detect homologous recombination.

### [Materials]

· Cells: *Pyricularia oryzae,* TG strain, RS strain
· Vectors: TG vector (pISTG-YFP:: BSD) and RS vector (pRS-YFP:: BSD-bar) (Yamato et al., Sci Rep, 9, 7427, 2019); Vectors for disrupting various genes (constructed based on pMK-dGFP: Arazoe et al., J Gen Plant Pathol, 79, 422-430, 2013, or pMK-bar: Arazoe et al., FEMS microbiol Lett, 352, 221-229, 2014); and a CRISPR/Cas9 vector (Arazoe et al., Biotechnol Bioeng, 112, 2543-2549, 2015)
· Potato Dextrose Agar (PDA) medium

### [Method]

### (Construction of Disruption Vector)

Sequences (about 1000 bp) homologous to the upstream and downstream regions of each of the target genes (genes to be disrupted shown in Tables 1 and 2) were inserted into the upstream and downstream of the hph expression cassette of pMK-dGFP, respectively, to construct disruption vectors (hygromycin B-resistant strain was selected, and was used for gene disruption of the RS strain). Further, similar homologous regions were inserted into the upstream and downstream of the bar expression cassette of pMK-bar to construct disruption vectors (bialaphos-resistant strain was selected, and was used for gene disruption of the TG strain).

### (Construction of Gene-Deleted Strain)

Each of the target gene-disrupting vectors and a CRISPR/Cas9 vector capable of cleaving the inside of the target gene were introduced in combination by the protoplast PEG method (Yamato et al., Sci Rep, 9, 7427; Arazoe et al., J Gen Plant Pathol, 79, 422-430), to obtain target gene-deleted strains derived from the TG strain or RS strain.

### (Recombination Examination)

The TG strain and the RS strain were subjected to dual culture on PDA medium. Fourteen days later, the hyphae in the boundary region of hyphae were punched out with a No. 1 cork borer, and were inoculated into PDA medium containing blasticidin S. Among the inoculated hyphal fragments, hyphal fragments showing blasticidin S resistance and YFP fluorescence were regarded to have undergone pseudo-sexual reproduction (homologous recombination), and the pseudo-sexual reproduction frequency (%) was calculated by "(the number of hyphal fragments that have undergone pseudo-sexual reproduction)/(the number of total hyphal fragments) × 100". The influence of each of the target genes on the recombination efficiency was evaluated by dual culture of different gene deletion strains or dual culture with the TG strain or the RS strain in the same manner.

### [Results]

The number and ratio of hyphal fragments that developed blasticidin S (BS) resistance as a result of the fusion of one of the TG strains and one of the RS strains are shown in the following tables.

**[Table 1]**

| Hybridization | Number of BS-resistant hyphal fragments/total number of hyphal fragments (ratio) |
|---|---|
| TG vs RS | 227/252 (90.1%) |
| TG vs RSΔham5 | 64/280 (22.9%) |
| TG vs RSΔste11 | 136/392 (34.7%) |

**[Table 2]**

| Hybridization | Number of BS-resistant hyphal fragments/total number of hyphal fragments (ratio) |
|---|---|
| TG vs RSΔspo11 | 103/252 (40.9%) |
| TGΔspo 11 vs RS | 46/112 (41.1%) |
| TGΔspo11 vs RSΔspo11 | 45/112 (40.2%) |

As shown in the above tables, it was found that the frequency of pseudo-sexual reproduction decreases by the deletion of hyphae (cell) fusion-related genes ham5 and ste11 and a meiosis-related gene spo11. This indicates that cell fusion and meiosis are involved in genome hybridization caused by pseudo-sexual reproduction.

Similarly, as a result of examining the frequency of pseudo-sexual reproduction after deleting other genes, it was found that the frequency of pseudo-sexual reproduction was also decreased by deletion of genes involved in cell fusion, nuclear fusion, or meiosis, such as symc, symb, kar5, pmk1, whi2, so, rad51, ddnm1, mak1, mak2, and rec8. From these findings, it was presumed that gene editing or hybridization can be achieved by introducing a factor involved in cell fusion, nuclear fusion, or meiosis into the cell(s) and artificially inducing meiosis through cell fusion.

### 1-2. Pseudo-Sexual Reproduction in Pyricularia grisea

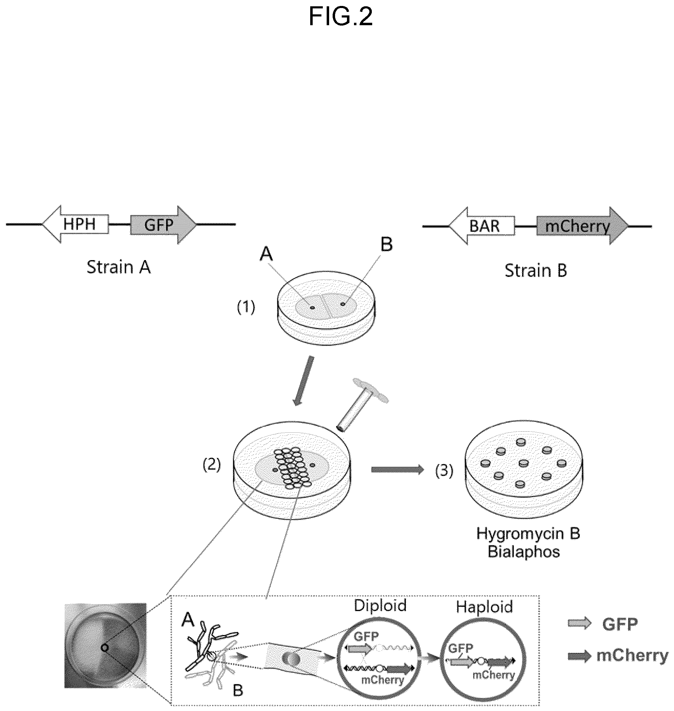

In the following experiment, a strain of *Pyricularia grisea* into which a GFP gene and a hygromycin B resistance gene cassette had been introduced (a GFP-Hph strain), and a strain of *Pyricularia grisea* into which an mCherry gene and a bialaphos resistance gene cassette had been introduced (an mCherry-Bar strain) were used as an experimental system for detecting genomic hybridization in pseudo-sexual reproduction. The outline of this system is shown in Fig. 2. In Fig. 2, the GFP-Hph strain is designated as Strain A, and the mCherry-Bar strain is designated as Strain B.

The GFP-Hph strain has a hygromycin B resistance gene cassette (Hph), a TEF promoter (PTEF), and a green fluorescent protein (GFP) gene. The mCherry-Bar strain has a bialaphos resistance gene cassette (Bar), a TEF promoter (PTEF), and a red fluorescent protein (mCherry) gene. When chromosome exchange occurs through a chromosome crossover reaction, a hypha that has undergone the chromosome exchange acquires dual resistance, namely, hygromycin B resistance and bialaphos resistance, and exhibits GFP fluorescence and mCherry fluorescence. Chromosomal recombination was detected using this phenomenon.

### [Materials]

· Cells: *Pyricularia grisea,* GFP-Hph strain, mCherry-Bar strain
· Vectors: GFP-Hph vector (pMK412 Neo) and mCherry-Bar vector (pMK-mCherry-Bar: the GFP gene of pMK412 Neo was substituted with the mCherry gene, and the Hph gene was substituted with the Bar gene)(Yamato et al., Sci Rep, 9, 7427, 2019); vectors for disrupting various genes (pMK-bar: Arazoe et al., FEMS microbiol Lett, 352, 221-229, 2014, or pMK-NeoR in which the Bar gene of pMK-bar had been substituted with a G418 resistance gene); and a CRISPR/Cas9 vector (Arazoe et al., Biotechnol Bioeng, 112, 2543-2549, 2015)
· Potato Dextrose Agar (PDA) medium

### [Method]

### (Construction of Disruption Vector)

Sequences (about 1000 bp) homologous to the upstream and downstream regions of the target genes (genes to be disrupted shown in Table 3) were respectively inserted into the upstream and downstream of the G418 resistance gene expression cassette of pMK-NeoR to construct disruption vectors (G418-resistant strains were selected).

### (Construction of Gene-Deleted Strains)

Each of the target gene-disrupting vectors and a CRISPR/Cas9 vector capable of cleaving the inside of the target gene were introduced in combination by the protoplast PEG method (Yamato et al., Sci Rep, 9, 7427, 2019; Arazoe et al., J Gen Plant Pathol, 79, 422-430, 2013) to obtain target gene-deleted strains derived from the GFP-Hph strain or the mCherry-Bar strain.

### (Recombination Examination)

The GFP-Hph strain and the mCherry-Bar strain were subjected to dual culture on PDA medium (Fig. 2 (1)). Fourteen days later, the hyphae in the mycelial boundary region were punched out with a No. 1 cork borer and were inoculated into PDA medium containing hygromycin B and bialaphos (Fig. 2 (2)). Among the inoculated hyphal fragments, hyphal fragments that showed dual resistance including hygromycin B resistance and bialaphos resistance, and that emit dual fluorescence including GFP fluorescence and Cherry fluorescence were regarded to have undergone pseudo-sexual reproduction (mycelial fusion, nuclear fusion, and chromosome homologous recombination at the time of mycelial contact) (Fig. 2 (3)). The pseudo-sexual reproduction frequency (%) was calculated by "(the number of hyphal fragments that have undergone pseudo-sexual reproduction)/(the number of total hyphal fragments) × 100". The influence of each of the target genes on the recombination efficiency was evaluated by dual culture of different gene deletion strains or dual culture with the GFP-Hph strain or the mCherry-Bar strain in the same manner.

### [Results]

The number and ratio of hyphal fragments that showed dual resistance and dual fluorescence as a result of the fusion of the GFP-Hph strain and the mCherry-Bar strain are shown in the following table.

**[Table 3]**

| Dual culture | Dual fluorescence/ dual resistance | Total hyphal fragments | Ratio (%) |
|---|---|---|---|
| GFP-Hph vs mCherry-Bar | 26 | 28 | 92.9 |
| GFP-HphΔMAT vs mCherry-Bar strain | 0 | 28 | 0.0 |
| GFP-HphΔSpo11 + mCherry-Bar strain | 2 | 28 | 7.1 |

As shown in the above table, it was found that the frequency of pseudo-sexual reproduction decreases by the deletion of the MAT gene region involved in mating, and by the deletion of a meiosis-related gene spo11. This suggests that mating-related genes, including meiosis, are involved in the genome hybridization caused by the pseudo-sexual reproduction.

### 2. Verification of Gene Editing by Introduction of Genes

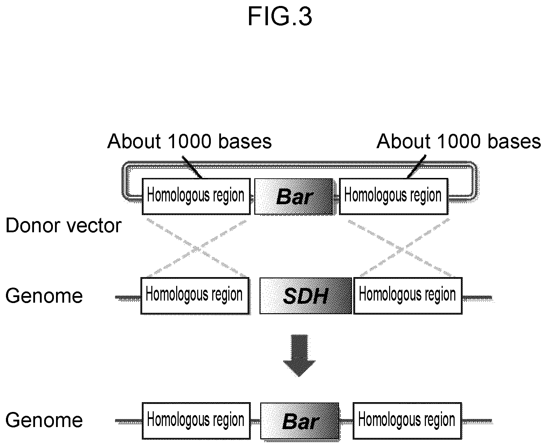

In this experiment, it is shown that gene editing using homologous recombination can be carried out by introducing various gene expression vectors into cells, and inducing the cells to undergo fusion. In this experiment, a gene-editing scheme was designed to replace the sdh gene, which is a melanin synthesis gene in the genome of *Pyricularia oryzae,* with the bar gene, which is a bialaphos resistance gene, using a donor vector. Strains that successfully underwent gene-editing acquire bialaphos resistance, and their colony color changes due to inhibition of pigment synthesis. The outline of the gene-editing scheme is shown in Fig. 3.

### [Materials]

· Cells: *Pyricularia oryzae*
· Vector: sdh gene disruption vector (Arazoe et al., Biotechnol Bioeng, 112, 1335-1342, 2015), promoting factor expression vector (pMK-dGFP: constructed based on Arazoe et al., J Gen Plant Pathol, 79, 422-430, 2013)
· PDA medium

### [Method]

### (Preparation of Vectors)

- Promoting-factor expression vector was prepared by cloning each of the genes shown in the following table into AscI and PacI sites of a pMK-dGFP vector located between a translation elongation factor gene (TEF) promoter derived from *Aureobasidium pullulans* and a glucoamylase gene (gla) terminator derived from *Aspergillus awamori.* Each of the gene sequences was amplified using the genome of the *Pyricularia oryzae* as a template and corresponding primer sequences (referred to as "primer for expression" below), to prepare promoting-factor expression vectors.

### Sequence of TEF Promoter, Cloning Site, and gla Terminator (SEQ ID NO: 13)

In the sequences, the sequence GGCGCGCC represents the AscI cleavage site; TTAATTAA represents the PacI cleavage site; the sequence upstream of the AscI cleavage site is a promoter sequence; and the sequence downstream of the PacI cleavage site is a terminator sequence.

5'-agcaaacggtggtcaaaggatggttcagatacaaattagcaacaggccaggctagacgcgcgactatccactgcgg caaatggtgagctgcaagcaacggtaagatgtgacaggacgagcggtgtgccgggaaaaaaattggaggagcgcaaagcggcgg ctgtccctcagtggtgcccaaacgttatcgatagtacaccaagcatgggcagtgagcggctatacagagggaataataggcatatcgg cacgactagattcggtagaaagcatcgaagagcaattcattgagcatattatcacgtggaatgcgatagctgtggccaggttgagacac cgcaagtgaaagatacacacatagattctcgattcgagcggtttgcctccgccaccgcagtgcatagcaagcaaagaaacgacagttg gctcatcatccgttacatcattttttctactggctccgctcggtgggctcccaacgaagcagcaaaaaagtgagagaaaaaaactagctt ggcggggcaacagaagctagaccctttggctcgcttagtcagtgcgcccactcactcacactcaaaaaggccacccctcccgcaccc tcttctcatcaccgtcttcataccacggttcgtcaagcaatcgtatctggtaagctttgacctcctcgagcgggctccactttgctatttcttg gatctgctctttcttttctctctacctctttttctaacctctcttcagaaagttcaaccgtacttcactcaatcttccatacacaaccgtcGGCG CGCCGCTAGCTTAATTAAacaatcaatccatttcgctatagttaaaggatggggatgagggcaattggttatatgatcatgt atgtagtgggtgtgcataatagtagtgaaatggaagccaagtcatgtgattgtaatcgaccgacggaattgaggatatccggaaataca gacaccgtgaaaggcatggtctttccttcgtgtagaagaccagacag-3'

### (Transformation)

A donor vector alone (negative control), or a donor vector and each of the gene expression vectors shown in the following table were introduced in combination by the protoplast PEG method. Among the colonies resistant to bialaphos, strains whose melanin synthesis was inhibited were regarded as strains that had been successfully edited.

### [Results]

The following table shows the total number of colonies (Total), the number of colonies that had been successfully edited (Positive), and the rate of successful editing (Efficiency), when each of the gene expression vectors was used. From the following table, it was confirmed that gene editing occurred by introducing a donor vector and HMG 1 ox, HMG2 ox, α-1 ox, α-2 ox, CD ox, CDA ox, Ung ox, SymC ox, Ste11 ox, Pmk1 ox, Mre11 ox, Rec8 ox, Ddnm1 ox, Spo11 ox, Pro1 ox, CDA ox and Ung ox, Mre11 ox, Rec8 ox, Ddnm1 ox, Spo11 ox, or Pro1 ox in combination.

**[Table 4]**

| Transgene | Total | Positive | Efficiency (%) |
|---|---|---|---|
| Donor vector (negative control) | 57 | 0 | 0.0 |
| *HMG1* ox | 10 | 4 | 40.0 |
| *HMG2* ox | 53 | 4 | 7.5 |
| *α-1* ox | 5 | 1 | 20.0 |
| *α-2* ox | 58 | 1 | 1.7 |
| *CD* ox | 65 | 1 | 1.5 |
| *CDA* ox | 60 | 2 | 3.3 |
| *Ung* ox | 18 | 1 | 5.5 |
| *SymC* ox | 36 | 2 | 5.5 |
| *Stell* ox | 42 | 1 | 2.3 |
| *Pmk1* ox | 8 | 1 | 12.5 |
| *CDA* ox + *Ung* ox | 25 | 3 | 12.0 |

**[Table 5]**

| Transgene | Total | Positive | Efficiency (%) |
|---|---|---|---|
| Donor vector (negative control) | 107 | 0 | 0.0 |
| Mre11 ox | 77 | 1 | 1.3 |
| Rec8 ox | 66 | 1 | 1.5 |
| Ddnm1 ox | 67 | 1 | 1.5 |
| Spo11 ox | 70 | 1 | 1.4 |
| Pro1 ox | 6 | 1 | 16.6 |

### 3. Verification of Recombination Between Heterologous Genomes

This study shows that homologous recombination occurs by artificially inducing cell fusion between cells with heterologous genomes, and that the introduction of a nuclease promotes homologous recombination.

### 3-1. Verification of Recombination Using Pyricularia oryzae TG and RS Strains

### [Materials]

· Cells: *Pyricularia oryzae,* TG strain, RS strain
· Vectors: pGEM-I-SceI (Arazoe et al., FEMS microbiol Lett, 352, 221-229, 2014), pMK-dGFP (Arazoe et al., J Gen Plant Pathol, 79, 422-430, 2013), and pMK-bar (Arazoe et al., FEMS microbiol Lett, 352, 221-229, 2014)
· Medium: PDA medium
· PEG solution (polyethylene glycol 4000, 50 mM CaCl₂, 0.25 ml, 10 mM Tris-HCl (pH8.0))

### [Method]

### (Genome Hybridization Examination)

Groups (A) to (D) were subjected to transformation in accordance with the following method. (A) and (B) are negative controls.
(A) TG + bar: the pMK-bar vector was introduced into 5 × 10⁴ protoplast cells using the protoplast PEG method.
(B) RS + hph: the pMK-dGFP vector was introduced into 5 × 10⁴ protoplast cells using the protoplast PEG method.
(C) TG + RS: a PEG solution was added to a mixed solution of 5 × 10⁴ protoplast cells derived from the TG and RS strains.
(D) TG + RS + I-SceI: pGEM-I-Scel vector was introduced into a mixed solution of 5 × 10⁴ protoplast cells derived from the TG and RS strains using the protoplast PEG method.

The protoplasts treated as described above were collected, and seeded on PDA medium containing bialaphos for (A), or seeded on PDA medium containing hygromycin B for (B). The transformation efficiency (%) was calculated based on the number of drug-resistant colonies obtained. For (C) and (D), the cells were seeded on PDA medium containing blasticidin S, and the genome hybridization efficiency (%) was calculated based on the number of drug-resistant colonies obtained. The seeded samples were cultured at 28°C for 10 days, and then colonies were observed.

### [Results]

The following table shows the number of hph or bar resistant colonies (Hpr or bar positive), the number of blasticidin S resistant colonies (BS resistant), and the transformation efficiency (Efficiency), in each group of cells. From the following table, it can be confirmed that, when protoplast cells are fused in accordance with (C), blasticidin S-resistant strains are generated by cell fusion and nuclear fusion followed by a meiosis-like genome hybridization reaction (homologous recombination). In addition, it can be confirmed that the number of blasticidin S-resistant strains obtained in (C) and the efficiency thereof are higher than the number of transformants (cells into which foreign DNA is introduced) obtained in (A) or (B) and the efficiency thereof. It can also be confirmed that, when protoplast cells are fused in accordance with (C) and (D), the recombination efficiency is significantly improved by introduction of the nuclease I-Scel.

**[Table 6]**

| Cell fusion | Hph or bar positive | BS resistant | Efficiency |
|---|---|---|---|
| TG + bar (transformation control) | 13 | - | 8.7x1 0E-5 |
| RS + Hph (transformation control) | 10 | - | 6.6x10E-5 |
| TG + RS | - | 131 | 8.73x10E-4 |
| TG + RS + I-SceI (nuclease) | - | 386 | 2.57x10E-3 |

### 3-2. Verification of Recombination Using sdh Gene-Disrupted Strain and GFP-Hph Strain of Pyricularia oryzae

### [Material]

· Cells: *Pyricularia oryzae,* sdh gene-disrupted strain (ΔSDH cells), GFP-Hph strain (GFP cells)
· Vectors: a CRISPR/Cas9 vector (Arazoe et al., Biotechnol Bioeng, 112, 2543-2549, 2015), pMK-dGFP (Arazoe et al., J Gen Plant Pathol, 79, 422-430, 2013), pMK-bar (Arazoe et al., FEMS microbiol Lett, 352, 221-229, 2014), HMG1 ox, and HMG2 ox
· Medium: PDA medium
· PEG solution (polyethylene glycol 4000, 50 mM CaCl₂, 0.25 ml, 10 mM Tris-HCl (pH8.0))

### [Method]

### (Genome Hybridization Examination)

Groups (A) to (D) were subjected to transformation in accordance with the following method. (A) and (B) are negative controls.
(A) ΔSDH cells + GFP: pMK412 Neo vector was introduced into 5 × 10⁶ protoplast cells of an sdh gene-disrupted strain by the protoplast PEG method.
(B) GFP cells + mCherry: the pMK-mCherry-Bar vector was introduced into 5 × 10⁶ protoplast cells derived from the GFP-Hph strain by the protoplast PEG method.
(C) ΔSDH cells + GFP cells: a PEG solution was added to a mixed solution of 5 × 10⁶ protoplast cells derived from an sdh gene-disrupted strain and GFP-Hph strain.
(D) ΔSDH cells + GFP cells + CRISPR: a CRISPR/Cas9 vector was introduced into a mixed solution of 5 × 10⁶ protoplast cells derived from an sdh gene-disrupted strain and a GFP-Hph strain by the protoplast PEG method.
(E) ΔSDH cells + GFP cells + HMG: HMG1 ox or HMG2 ox vector was introduced into a mixed solution of 5 × 10⁶ protoplast cells derived from an sdh gene-disrupted strain and a GFP-Hph strain by the protoplast PEG method.

The protoplasts treated as described above were collected and seeded on PDA medium containing hygromycin B and bialaphos, and the number of colonies obtained was calculated. The seeded samples were cultured at 28°C for 10 days, and then colonies were observed.

### [Results]

From the following tables, it can be confirmed that, when protoplast cells are fused in accordance with (C), a melanin biosynthesis-deficient GFP fluorescent strain in which two traits are mixed is generated by cell fusion and nuclear fusion followed by a meiosis-like genome hybridization reaction (homologous recombination). In addition, the number of transformed strains obtained from (D) and (E) is larger than that obtained from (C), suggesting that the recombination efficiency is improved by genomic cleavage via a nuclease or by promotion of the meiosis-like reaction.

**[Table 7]**

| Protoplast fusion | Total no. | Hybrid no. |
|---|---|---|
| ΔSDH cells + GFP vector | 85 | 75 |
| GFP cells + mCherry vector | 34 | 28 |
| ΔSDH cells + GFP cells | 6 | 6 |
| ΔSDH cells + GFP cells + CRISPR/Cas9 | 17 | 17 |

**[Table 8]**

| Protoplast fusion | Total no. | Hybrid no. |
|---|---|---|
| ΔSDH cells + GFP vector | 134 | 127 |
| GFP cells + mCherry vector | 24 | 17 |
| ΔSDH cells + GFP cells | 2 | 2 |
| ΔSDH cells + GFP cells + CRISPR/Cas9 | 5 | 5 |
| ΔSDH cells + GFP cells + HMG1 ox | 8 | 8 |
| ΔSDH cells + GFP cells + HMG2 ox | 3 | 3 |

### 4. Verification of Gene Editing in Aspergillus sojae

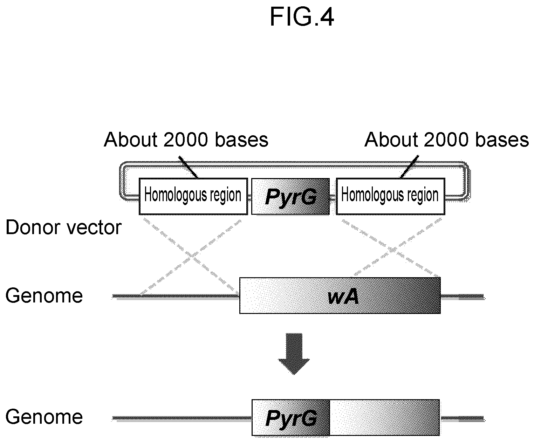

In this study, it is shown that gene editing by homologous recombination can be carried out by promoting cell fusion and a subsequent meiosis-like reaction in *Aspergillus sojae.* In this study, a gene editing scheme was designed whereby the wA gene, which is associated with pigment synthesis in *Aspergillus sojae,* was replaced with a uridine/uracil biosynthetic gene pyrG, using a donor vector. Strains that have been successfully gene-edited acquire uracil auxotrophy, and have whitened conidia due to inhibition of pigment synthesis. The outline of the gene editing scheme is shown in Fig. 4.

### [Materials]

· Cells: Uracil-auxotrophic *Aspergillus sojae* (ΔpyrG strain, MAT1-1 type)
· Vector: *Aspergillus sojae-derived* MAT1-1 (AsMAT1) expression vector, *Aspergillus oryzae-derived* MAT1-2 (AoMAT1) expression vector, wA gene disruption vector (donor vector)
· Medium: Czapek-Dox medium
· PEG solution (polyethylene glycol 4000, 50 mM CaCl₂, 0.25 ml, 10 mM Tris-HCl (pH8.0))

### [Method]

### (Construction of MAT1 Gene Expression Vectors)

A MAT expression plasmid was prepared as follows, in which Ptef, which is a promoter sequence of the translation elongation factor gene tef1 (748 bp upstream of the tef1 gene, SEQ ID NO: 1); the MAT1-1 gene derived from *Aspergillus sojae* (SEQ ID NO. 2) or the MAT1-2 gene derived from *Aspergillus oryzae* (SEQ ID NO. 3); and Talp, which is a terminator sequence of the alkaline protease gene alp (800 bp downstream of the alp gene, SEQ ID NO: 4), were linked to the plasmid pUC19.

DNA fragments of Ptef, the MAT1-1 gene derived from *Aspergillus sojae,* and Talp were amplified by PCR using chromosomal DNA of the *Aspergillus sojae* NBRC4239 strain as a template, and using primer pairs of SEQ ID NOs: 5 and 6, SEQ ID NOs: 7 and 8, and SEQ ID NOs: 9 and 10, respectively. The MAT1-2 gene derived from *Aspergillus oryzae* was amplified by PCR using artificially synthesized DNA as a template and using primers of SEQ ID NOs: 11 and 12. In the multiple cloning site of pUC19, amplified Ptef, each of the MAT genes, and Talp were ligated using In-Fusion HD Cloning Kit (Clontech Laboratories, Inc.) in accordance with the protocol attached to the kit, to obtain MAT expression plasmids. As the pUC19, the pUC19 linearized Vector attached to the kit was used. 
SEQ ID NO:1 (Ptef): tgtggaccagacaggcgccactcggccgggccacaactgcttgggttttgaccgggagc
ggaccaattaaggactcgaacgaccgcggggttcaaatgcaaacaagtacaacacgcagcaaacgaagcagcccaccactgcgttg
atgcccagtttgtctgtccgaaatccaccggaaaggtggaaacatactatgtaacaatcagagggaagaaaaattttttatcgacgaggc
aggatagtgactgatggtggggtcatggtcgggtctccgagcgaaagagaaccaaggaaacaagatcaacgaggttggtgtaccca
aaaggccgcagcaacaagagtcatcgcccaaaagtcaacagtctggaagagactccgccgtgcagattctgcgtcggtcccgcacat
gcgtggtgggggcattacccctccatgtccaatgataagggcggcggtcgagggcttaagcccgcccactaattcgccttctcgcttgc
ccctccatataaggattcccctccttcccctcccacaacttttttcctctttctctcttcgtccgcatcagtacgtatatctttcccccctacctct
ttctcactcttcctcgattcattccactcttctccttactgacatctgttttgctcagtacctctacgcgatcagccgtagtatctgagcaagctt
ttttacagaatctttctagtatcttacaaagaactacaaagttcgcaccaccttcaaa
 
SEQ ID NO:2 (MAT1-1 gene): atggaaaccacagtgtctcccctccagcgtgcgtttaacgcattcttgatga
ccatgccacccgagcagttggaggagctgctcaagtacctccaggacgcaaaatctcaggaaaatactcaatcttcatatccgaaaga
aaatcttcaatcttgcttggagttcaaggcagacaaaaacaatggatctaccaaccctgcgagtgctaacccacggtcctcagcttcgcg
ggggaagcgtgcctcagatgcaaaacgaaggccgctcaatagtttcattgcctttaggagtgagtgtccttgtaccgtgatcgagcgaa
ttccacattctaacaatactaggctattactccatcatgttccctgacctcactcagaaggcaaagtcgggcattcttcgcttcctgtggcag
aacgatccattcaaagccaaatgggcaatccttgccaaagcctactccatcgttcgcgatgaccacgatagtgatgtttctctggaatcttt
tttgggtttgaatgcacagttcattggtataattggaccaagtcgttatcttgaagttatgggctggcaactggatgtcgacgatcagcaac
aatatactatagctagggttaaagcgacgactgcaaatgaagccgatatttcgaccaactattctgtcaatgatatagttaaacattgctac
 
accagtggctatgtttccgagaaaaatcggaagagcaaggcaagcaataacaacagtgccccggttatggcttttgctgcccagccga
ccttggttgtccacaagaacgacagtattcgggtatctggcaaccatactattgtaaccgatgtttacaaaacgaatccagctatggaaat
atcttcaccggagcaaactgaagatacattttcgccaaacactagtgacttgagcacaattgctgatgaacctccgctcgatgcaatgga
agtagtgggtatctgcaaccgccctcagctttactcggattcaagtacgccgaattgttttgatttcgataacatccaattcccaggctggg
atgaggagaacgcaatgttcacttacgatgccgcactacaaacgccactgatgccctatgatccgctgaactacgatccactggaagcc
tatgacttctctagattcgttgatatttga
 
SEQ ID NO:3 (MAT1-2 gene): atgacgactatacctatcgctatgaagacgaccgcggagtcgacggacaa
gcttacagagctgctttggcaggatgctttgcgtcacctcgagtctacaaataatgaggtgcttttgccgattaatgtcacagacatgatag
gtcaaagcaatgtcgacaagattaggactcgtctcgggtaagaagagttctcagattctctaggtatacaaacttacatttggtgatagtgc
tcttatcggtgctcctgtggtggcttttgtcgatgagacgatcaatgctctccgtgtaatgcgcacgcctgctttttcgggctccgttgtgtcc
gtcgcatctcatgacagaatttcgaacttagaaaaggaaatcaccgaagcatctggtagaacacatggcaaatccgcactaccaacga
agtccaaagtccctcgaccaccaaatgcattcatcctttatcgtcagcatcaccatcccagaatcaaagaagcatatcccgatttcaccaa
taatgagatatgtaagtttccttttcatctctactcaacttcgaacaagctaacgacatcagcaatcatccttggaaaacaatggaaggccg
aatccgaagaggtcaaaatgcaatttcgcaatatggcggagaaacttaagaaaaagcatgcagaagaccaccccgattaccattatact
ccccgcaagccatccgaaaagaagcgtcgagcttcgtcccgccagtactctaagccgaccaaacgacagaagtccccggccttgac
aaatgatacctctgatagctccacaccgtcgatgtactccggcatgcagctggacaacatacctgtagatgcatctttggataatttggct
gatatcgacatcgtgctcagccccgatgaactgcctagggactgtgggctacaattcgactcagttgccttcgataattttctccagcaag
tacagggcgactgtggcaagacagccgctactttgttcccacagttcaacttcacagaaaggcctgtgggagagtcattcgaattttcag
atttgattgctgactgctactga
 
SEQ ID NO:4 (Talp): gtaccaggagtacattggagagttctaccattgttgctggaatacaatgatgattagaaacc
gaagagtgttatgattcggacggatatacgcatggcacgcatacagcgtgatacataggctgtttgctcaagaattaggattttatctgaat
ccatgtacagagtttacttatgttagtagtcaatgaaatcttggctttctaattttgtccgatctacaaggggtagtcgatcacagaacgaact
agatgtgcagggaacgatgatcacccgctcttagcaagacctctagtagttttcgaccatagctttaacgcgaatcatgaccctactatttt
ctagattgcagaccaagtcacatgacaatgtcctctttgaagtaggatcagtagctgattagattccgggaaatgaattagggctggcgtt
ccaactactggggagtgccgatgttgctgtatgaaagatagtaagattactagtgcacagctgtagtaattatttactctagattatatattcc
aaataataagtaatctaagatagtagacagtcctatgatatagctccgggttcgaagtcggcaaaagatatgcaatcacctgtcgggatg
atatatgtatatctgaaataccgacatcaaccatccagtcggatcagctaaacgaagtatcacttctttcgccactgccaatcactacttcta
ttaaagttcatgttacagtataagccacaagacttatctccagaactaacttgtgcataggagctctgccgatagccgggtggttggatcg
g
 
SEQ ID NO:5 (Forward primer): cggtacccggggatcTGTGGACCAGACAGGCGCCA
CT
SEQ ID NO:6 (Reverse primer): TTTGAAGGTGGTGCGAACTTTGTAG
SEQ ID NO:7 (Forward primer): CGCACCACCTTCAAAatggaaaccacagtgtctcccctcc
SEQ ID NO:8 (Reverse primer): ATGTACTCCTGGTACtcaaatatcaacgaatctagagaagtc
SEQ ID NO:9 (Forward primer): GTACCAGGAGTACATTGGAGAGTTC 
SEQ ID NO:10 (Reverse primer): cgactctagaggatcCCGATCCAACCACCCGGCTAT
CGG
SEQ ID NO:11 (Forward primer): CGCACCACCTTCAAAatgacgactatacctatcgctatga
SEQ ID NO:12 (Reverse primer): ATGTACTCCTGGTACtcagtagcagtcagcaatcaaatct

### (Transformation)

The donor vector alone (negative control), the donor vector and the MAT1-1 expression vector derived from *Aspergillus sojae* in combination, or the donor vector and the MAT1-2 expression vector derived from *Aspergillus oryzae* were introduced or co-introduced by the protoplast PEG method. Among the colonies that had been supplemented with uridine/uracil auxotrophy, strains that had lost pigment synthesis in conidia were regarded as strains that had been successfully edited.

### [Results]

The following table shows the total number of colonies (Total), the number of successfully edited colonies (Positive), and the rate of successful editing (Efficiency), when each gene expression vector was used. From the following table, it was confirmed that gene editing was promoted in *Aspergillus sojae* as well by co-introducing the MAT1-1 expression vector derived from *Aspergillus sojae* or the MAT1-2 expression vector derived from *Aspergillus oryzae* with the donor vector.

**[Table 9]**

| Transgene | Positive | Total | Efficiency (%) |
|---|---|---|---|
| Donor vector (negative control) | 31 | 160 | 19.4 |
| AsMAT1-1 ox | 14 | 26 | 53.8 |
| AoMAT1-2 ox | 23 | 55 | 41.8 |

### 5. Verification of Promotion of Meiosis-Like Reaction in Human Cells (HeLa Cells) by Cell Fusion

In this study, it is shown that cell fusion of human cells with PEG promotes the meiosis-like reaction in a manner similar to filamentous fungi. In this experiment, the gene expression levels of the meiotic initiation factor (MEIOSIN), which is transiently expressed at the time of the switch from somatic division to meiosis, and of RAD51, which is involved in chromosomal recombination during meiosis, were measured by real-time PCR.

### [Materials]

· Cells: HeLa cells
· PEG1500 (MERCK)
· DMEN medium (NACALAI TESQUE, INC.)

### [Method]

### (Cell Fusion with PEG)

5 × 10⁶ cells washed with PBS were collected by centrifugation and tapped 2 to 3 times. Thereafter, 100 µL of a PEG1500 solution was added, and the cells were cultured while gently shaking at 37°C for 90 seconds. Thereafter, 300 µL of DMEN medium was added, and the cells were cultured again while shaking at 37°C for 90 seconds. Thereafter, DMEN medium (NACALAI TESQUE, INC.) containing 1 mL of 10%FBS was added thereto, and the cells were cultured while shaking at 37°C for 8 minutes. Using the total RNA extracted from the recovered cells, real-time PCR was carried out using primers targeting MEIOSIN and RAD51. The ACTB gene was used as an internal standard for gene expression, and cells to which PBS was added instead of PEG were used as a negative control.

The sequences of primers for HeLa cell real-time PCR are shown below.
Rad51-1 primer (SEQ ID NO: 14)
   5'-attgtatctgaggaaaggaagaggggaaac-3'
Rad51-2 primer (SEQ ID NO: 15)
   5'-ttttaacagaggaaaaacccaatgattcagtcttt-3'
MEIOSIN-1 primer (SEQ ID NO: 16)
   5'-ccagcgaagaggacgaagacaccacatgga-3'
MEIOSIN-2 primer (SEQ ID NO: 17)
   5'-atgaagccgttgacacacttcttcttcatctgggg-3'
ACTB-1 primer (SEQ ID NO: 18)
   5'-CAGCCTTCCTTCCTGGGCAT-3'
ACTB-2 primer (SEQ ID NO: 19)
   5'-CATTGTGCTGGGTGCCAGGG-3'

### [Results]

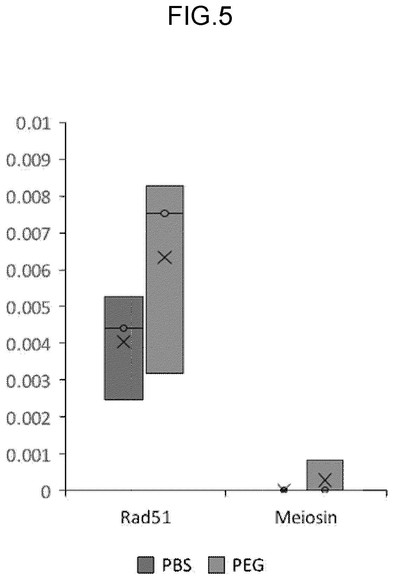

Fig. 5 shows the results of real-time PCR. By cell fusion with PEG, the expression of MEIOSIN, which is expressed specifically at the meiosis stage, was confirmed. In addition, the gene expression of RAD51, which is involved in the chromosomal recombination reaction during meiosis, was increased. These results indicate that a meiosis-like reaction also occurs in the process of cell fusion in human cells.

The gene sequences of *Pyricularia oryzae* and *Pyricularia grisea,* and the expression primer sequences used in the Examples are described below (data not shown for some of the genes). Of the following genes, the genes designated as *"Pyricularia grisea"* are genes of *Pyricularia grisea.*
*Pyricularia oryzae* MAT1-2-1 (MAT hmg1) (SEQ ID NO:20) 
5’-atggattcattgcagatggactcctctcaaatggaggcgttgtcgtcgatttgggtcatcgtgcagcatcagctcgcccaaatcaaca
attccaacaacatagctcatatcggcgccgttctcctcaaccaacttgaggcgcctttcaggagcactttgacatcatggtttcagcagta
agttctccataagctcaaggcttttccgcaagggaaactaacatcaggtaggcacaccggcatcaaccaggtctcagtcgtgcgcgac
atcaaggctcgtcgacactacatcggaccaactgatgcttttcaggagagctatcaagttgtacacatccagggtcacggatttgtctgg
ctttgggatctccaggttgatggtgaggatgccagcatcaattcctgcaatatctctgccgagacagcacagaacttttcccagctgcgtc
ttgacgagatggcgcctaccaatgatgttacgatggtaggtgatgagagcctcatcaacggcaacggcattgactttgccatgttggaa
gacatcggaagccccgcatcaaacggctcctcattcccgagccctcaagttttcacccgggaaatttcattcgatgatctaccagttggc
attatgactgccgaggccattcagagagaccttcttggcgccaatattcgcccggctaacctcattgatccgagtcttgcgagcaatcag
aatgtccacaacatcatccatcagaataacattgcccaggcttttacagatcacctgaacatgatggcatccaatgctgtccacgatgaca
cagaagagggtctgacagccgcggatcttatgccggcgttgcatcgcatgttcgtgccgggtcttgagtcaattgcaatggagcagttc
cacaccacgcttcctaatggcgcactccttcacctgtcaattcctgtcgatgaacctgaggatgctgtctcgaccaccgcgcctgctgctc
aacagacttccttgcaagacaagatccctcgtcctgcgaatgcctacatcctgtaccgcaaggattggcatcccatagtcaagtctgcaa
atccaggaatccataacaacgaaatctgtgagttcttttacctccaaacttccctagctgctgctaactttttttcctttagcaaaaatcctggg
gaagcagtgggcagctgagacaccggaagtgcgggctgagtacaaggagctcgcggaggagaagagagagttctacgcaaa
gtaccctacatatcgctactctcctcgtcggccgagtgagataatgcgccgagtgagtttgtaacacattattgcgtgaaagagtggaca
gcgagctaacaaattacttgccaacaagaacacgaaaagccgaaagaccccaacgaacggggcagcgaagccgagcaaagcaaa
gaagtgcggcaacggggttactggtggagcagacagtgcagtcgatgtcagctccccgggatcgccattttag-3’
Expression primer sequence 1 (SEQ ID NO:21)
   5'-gtgaatggtttaattaactaaaatggcgatcccggggagc-3'
Expression primer sequence 2 (SEQ ID NO:22)
   5'-cgaggcaggcgcgccatggattcattgcagatgga-3'
*Pyricularia oryzae* MAT1-2-2 (MAT hmg2) (SEQ ID NO:23) 
5’-atgccggccctctggtcaccagtcaaagtttgcggcatcgtttcggagctcaggtaagccgacccagagtcctcacaccagagcg
acagctcttcagcatctaacgatcacaggctaacacttttggcgcctcaattagcaagaacatggcctgcatggtttggatctgggtcgtc
tgcgttcctcagaagcggtaccgcatcacgacagctcgctggaactccagtgggctgctggttggccgcgttgattgtgagttttctactt
 
tcttcaccttttccttccgtcccagatctgagcagagactgacaagcgacacaaaacctccccacagctcctcctcaatctgccaccaca
gcgtcctcacagtcatcgtcattcgacggcagtgaggaagacacaaacacaaacatcaacgccaatggtatcgatgagagcctgctgc
cgcctgctctccgcggccgcactggaccttccaagcctccaaaccgctggattctctaccgtgccgccaagtcagctgagctgcgggc
agacaacccatccatgaacgcgagcgaaatctgtaagttcttcgctcttttcactctttttctccgctaccacttgtacctaccttttccccatc
tcccatactctttgaaaagtctccagtcccacctctcaagctgctgttttgacaagataaaagactaaccaacatctctctcctcacacagct
caagttgcatccctgatgtggcaggcagagtccgccgcgacaaaggcagagtgggaggagcgcgcagccgaagcccgcgagga
gcacatgcgaaacactccagagtatgcggtggcggccggtctgcgccagcgcgccatcgccgagaggagaaggcagaggcagca
gaagaagaagcaacaggagcagcagaagggcgacggtgacaaggtggccaaggagacttctacatccacagagagcttggtcact
acggacgtcggcgctgtcagcaaccacggcagcagcagcagtgaggtgcctgtcacaagcccacactcgccgacactactcagtcc
cgcgtctgacagcgattgtacgcccgtcggcagccctcgagctcgccacggctggggttctgatgctctcagcctgatgctcgacgcc
gccgaattctctactctcttctga-3’
Expression primer sequence 1 (SEQ ID NO:24)
   5'-cgaaagaaggcgcgccatgccggccctctggtca-3'
Expression primer sequence 2 (SEQ ID NO:25)
   5'-cagagtgccagttaattaatcagaagagagtagagaattcggcggcg-3'
*Pyricularia oryzae* MAT1-1-1 (MAT α-1) (SEQ ID NO:26) 
5'-atgattgccagtctctcgcctgacgacatcgccaggctgattccccaggagactctgacttctctgctcagagcaaatgacgagaaa
gagcgcctgcgggagctgcccgttagcccgagagcggtagcagcggcgagcaaaaacaagaagaaagtcaatggatttatggcatt
tcgatgtgagtacagactgataatagagaaatcgaagcttgtgctgacagcagtatagcctactacgctggcatctttcaggatagaccg
cagaaagaacgttcgcccttcatcactctactctggcagaaggaaacgctcaagtcaagatggactctcatggccaatgttttctctcgca
ttagggactttgcaggcaccacaagaggtcgtatggcaatgagtggattcctgcgagttgcctgcccgcttctgggcatcaccaaaccc
tgcgactacctcagacgctacaattgggaactcgagtttgttgcagacgcgtcagctccgtatgatgctgctatgaaatacgagatttccc
agtcacaaatccctcacattgtcgacgaattcgaggtccctactacggagattgagttgcttcgcgcctgcgttcagggtggctttccattt
gagaacagtgcccagcttctaagagacatggaagacagctctgtcaccgttatgaccagaactgcgcctatcatggcaccctcgcatg
cttcgcaggcctcgcatggccaacataaccaccatttcatcaacacactcatcaacgacccggatgctgcgatatctgcgcttttgcccc
aagatgaagacattggcagtctgatggttgacatgaatatcatccactcactcgagacagacagctcaacaacatcatcggcacgcaac
tcagtttctcctctggagcagcacttgttctttcacgaagacgtctccatcgatccttcaacaatggtttctttccctggcgaaggtcatggtc
acccggaaacacaatacagctaccccaatccaacgctaggtctctggtaa-3'
Expression primer sequence 1 (SEQ ID NO:27)
   5'-cgaaagaaggcgcgccatgccggccctctggtca-3'
Expression primer sequence 2 (SEQ ID NO:28)
   5'-cctgagatttaattaattaccagagacctagcgttggattgggg-3'
*Pyricularia oryzae* MAT1-1-2 (MAT α-2) (SEQ ID NO:29) 
5'-atgtcaactgcatcagattatctatacataaagattcctcccttcaacttctacaagaatggaaaaccagagccagaggatattgctgt
ctatcgagcgcgcccgtttgtatctgcatgcaagacccttctcggttctgtcgcggaactcttaggcgatagcagcatgaaccaaaacac
cgagtgccatcaagccattgtctctgctttgcaagcaaccaaagagttttttaccctgcaagcattattagacacactgaaggcttacaaa
gacggctctgagcctcttccaaagaagtggacagagcagagcctgcagggtgctttacaccgagcccgatgcgccatacgtgattca
gatcacgcccgaaagctcagctatgctatttcgctttgcttcgatttatctcagcgaaccaaggttaactcaggcaatacctgtcttctggc
cgccgtggtgacccttctggcaatgagggtgcggacactccagtatgagccagttgctcagtctgtcagcgcttctattggccttctcag
ggtccaagatggttgccttgttctcagagagcacagtcatcctaaggactacgggcaatggcttgatattatcgagcacccctctcttcca
acaatgccgggcagccgatggggcctgtttcatcgggtcctagacctgcctttcttcgatcaagaaaagctgcgcgattttactttcataa
caactcactgcgccaactccttgatacgttctacaatggggtgctacactaccgcacgtcagcaagctgctacactgcgtgaaagcgtc
atacgcttgagctatgaagatgagcaggtatgcactctaacatgtccctgtatttacactgctaactttgagaagaccaagataaagattgc
aaatgatcgcgcgcggctacctctaaatcctccagtggtaggcttgttgactcttctattttga-3'
Expression primer sequence 1 (SEQ ID NO:30)
   5'-cgtcaggcgcgccatgtcaactgcatcagattatc-3'
Expression primer sequence 2 (SEQ ID NO:31)
   5'-gtaatgttttaattaatcaaaatagaagagtcaacaagcc-3'
*Pyricularia oryzae* MAT1-1-3 (MAT α-3) (SEQ ID NO:32) 
5’-atggactacgagaacagcatagtggcgagccagatgcctgcagagaggatatctgtcagcgtctcgatacctcaacaaacagttg
atttcgaggtgccggccctctggtcgctaggcaaagtttgcggcatcgcttcggagctcaggtaagccgacccagagtcctcacacca
gagcgacagctcttcagcatctagcgatcacaggctaacacttttggcgcctcaaattagcaagaacatggcctgcatggtttggatctg
ggtcgtctgcgttcctcagaagcggtaccgcatcacgacagctcgctggaactccagtgggctgctggttggccgcgttgatcgtgagt
tttctactttcttcaccttttccttccctcccagatctgagcagagactgacaagcgacacaaaacctccccacagctcctcctcaatctgcc
accacagcgtcctcacagtcatcgtcattcgacggcagtgaggaagacacaaacacaaacatcaacgccaatggtatcgatgagaac
ctgctgccgcctgctctccgcggccgcactggaccttccaagcctccaaaccgctggattctctaccgtgccgccaagtcagctgagct
gcgggcagacaacccatccatgaacgcgagcgaaatctgtaagttcttcgctcttttcactctttttctccgctaccacttgtacctacctttt
ccccatctcccatactctttgaaaagtctccagtcccacctctcaagctgctgttttgacaagataaaagactaaccaacatctctctcctca
cacagctcaagttgcatccctgatgtggcaggcagagtccgccgcgacaaaggcagagtgggaggagcgcgcagccgaagcccg
cgaggagcacatgcgaaacactccagagtatgcggtggcggccggtctgcgccagcgcgccatcgccgagaggagaaggcagag
gcagcagaagaagaagcaacaggagcagcagaagggcgacggtgacaaggtggccaaggagacttctacatccacagagagctt
ggtcactacggacgtcggcgctgtcagcaaccacggcagcagcagcagtgaggtgcctgtcacaagcccacactcgccgacactac
tcagtcccgcgtctgacagcgattgtacgcccgtcggcagccctcgagctcgccacggctggggttctgatgctctcagcctgatgctc
gacgccgccgaattctctactctcttctga-3’
Expression primer sequence 1 (SEQ ID NO:33)
   5'-tcatcgtggcgcgccatggactacgagaacagcata-3'
Expression primer sequence 2 (SEQ ID NO:34)
   5'-gtgccagttaattaatcagaagagagtagagaattcggcgg-3'
*Pyricularia oryzae* rad51 (SEQ ID NO:35) 
5’-atgacgcaggttgaggatgactacgatgagagcgccaatgcggatgagagcggcggggctggtccaggagcacctacgcctct
gactgctttggaggtatgctttgcccgataaactccgtagaagtcgtcctcgctaatgtattgatgaaatcattcagggcgttggaggccta
acaaagagggatattcaactggtcatggaaggtggatacaacaccgtagagtctgtcgcctacacacccaggcggatgttggagcaa
atcaaaggaatctcggaacaaaaggcaggaaagattctcgcagaaggtaagatgagttgatgtgatacaaccagaatgacctgcctca
agcctcgtcactaaccacaccctgtcaatagcatccaaacttgtacctatgggcttcacaacggcgactgagatgcaccagcgcagaa
gtgagctgatatcgatcacgactggttcgaagaacctcgataccatgctgggtggaggtatcgagaccggctctgtcacggagctcttt
ggagagttccgtacaggaaaatcacagatttgccacacgcttgccgtgacatgccagttgccctttgatatgggcggcggagagggca
aatgtctatacatcgacaccgagggcacattccggcccgttcgactgctcgccgtggccaaccgctttggattgtcgggcgaggaggt
gctcgacaacgttgcctacgcacgcgcgtacaactccgaccatcagcttcagcttctgaaccaggcttcggccatgatgtgcgaaacg
cgcttcagtctccttattgtggattctgccaccgcactgtaccgtaccgacttccttggtcgtggagagctgagcagtcggcagactcatc
tagcaaagttcatgcggcagctgcagaggctggcggatgagtttggtattgcagttgtcatctcaaatcaggttgttgcacaggtcgatg
gtggtccctcggccatgttcaaccctgatcccaagaaacccataggcggtaacatcatcgctcacgccagcactaccagactgagtctg
aagaagggtcgcggcgagaccagaatcgccaaaatttatgacagtccatgtctaccagagagtgactgtctattcgccatcaatgagg
acggcattggtgacccttcgccgaaggatgtcgagaagatgaacaactga-3’
Expression primer sequence 1 (SEQ ID NO:36)
   5'-aaaggcgcgccatgacgcaggttgaggatgactac-3'
Expression primer sequence 2 (SEQ ID NO:37)
   5'-aaattaattaatcagttgttcatcttctcgacatc-3'
*Pyricularia oryzae* ddnm1 (SEQ ID NO:38) 
5'-atggcttctttacgaggcatgtgggctccagcagccgtacgtgtccttcgtcaggccgtcggcagtactgtcaaaatatcgaaagtg
ctgcgcaccaagctgggaagcgcagcacaccgaccgatcacccagcttcctcagcctgttacatctgcagctaggcaccaacccatc
cacccattcgcatttctgcgccagcagaaacgcggtttccgatggcactccgcccttcgcaacactcactatgatgccgtacgaaacac
ccttcgtcgcttcatcagtgggtcagcacgtgcccagccttcgcagtcgggccagacaacacgctgggttaagcagttcacaggtcgt
gctccttttgcgtcgacgctgcgtccgaacttgaccggaggtgcattcccgagaaccgccggcgggtatgcgatgccaggatctggaa
agatgggtggtgcacgctatttcagccatgcaccccctgtgcaagctcaggttatgcaaaatgttgggcaggcaatgcgggctttctgg
ctctcgggccagcgcgcgcactttgacggatatgggcctcggggagagaagtgctatcgtgtcaccaccgagttgcaggacgagac
cgcgaggaaggccgcatcagctgctgcatcacacaagcgtgtgccgggttccttcattgacttttctgtcaatcctaccatcacggcact
cagcccccttgccggtaacttccccttcgctatgcgcggtatcgaacaagaaaccgcagtcaatggccccacgttgaaccaagatggg
ctcctcgatctgctcttgaccgatttccagcgtgcgttcaaggatcttaatgctgtcatggcagatctaaagaggctctcaaatcttggaga
tctaccaatcgtcttcgagaatgattctcaccttgtacgtgttcggttcccaggtgtggatgcggatactgtaacgaggctctgcgaagata
 
ttggcatccaacaggggattgttggacaggacgaagacttcaagacggcccaggactatccatcggacctgcgcttcccatttgcgcct
gaccacggcaatccaaaccacttatcttcgcctggtgggtctttgagatctcatcaatcctactcatcagatctgtcaattgatttggatgat
gagtatgtggagcattttatggaggagaacccttggctctcctcagtagatgcagatgaatataaccattctcgcatcagcctagtctcac
ccactccggacaggcaaatcaactcacagttacaggacttcgagggcgtggaaggcatttatcgatttatacaagagtgtgatcgagct
gctgggcgtctttga-3’
Expression primer sequence 1 (SEQ ID NO:39)
   5'-gtcggcgcgccatgacatccccaaacaacccccca-3'
Expression primer sequence 2 (SEQ ID NO:40)
   5'-gtttaattaactacggcccgggggcagggcgagta-3'
*Pyricularia oryzae* CD (SEQ ID NO:41) 
5’-atgacatccccaaacaaccccccagaccacaaagccctcatggcccaagccctgaccctcgccagatcctcgccgtgcctaccc
acaaactaccgcgtcggcgctttgctagtcgacccatccccttccacagcatcatcacaagaaccctccaccccctcctcccccacagt
cctcgcaacaggctacaccctcgagctcccaggcaacacccacgcggagcagtgctgcatcgccaaggtctcctcgccagcagccg
acgcaccacccagcaccgagctcccaggcgaccagcaaatacagcaactagccctgcccaggggccggctggccctgtacacgac
catggagccgtgcgtggagcgcctgagcggcaacctgccgtgcgtgcagcgcatcctcgcctaccgggactttatcaccgacgtttat
gtgggcgtgctcgagcccgacacctttgtcaagggcaactcgggccgcgccgtgctagaggccgctggtgtcaaggtgcatcacgtc
gttggtctcgaggatgagattctcgaggttgcaaccgccggccatactcgccctgcccccgggccgtag-3’
Expression primer sequence 1 (SEQ ID NO:42)
   5'-gtcggcgcgccatgacatccccaaacaacccccca-3'
Expression primer sequence 2 (SEQ ID NO:43)
   5'-gtttaattaactacggcccgggggcagggcgagta-3'
*Pyricularia oryzae* CDA (SEQ ID NO:44) 
5’-atgtcgctcccgccctcgatccaaaagctgagcagcagcgacgacgcggcggtggatgaagtctgcaagacgtacggcatcag
cagggagcagttccagcagttgcaacgccgcagcgaggcggcaaagtcgacggcctactgcccctacagcaacttccgcgtcggc
gccaccctgctttcaatcgacgacaagaactcgtacataagcggcgccaacgtcgagaacgcatcgtacccagtcggcacgtgtgcc
gagcgcgtggcccttggcaaggccgtcaccgagggtcaccggcgattcagggccctcgcggtgtccacagacatcactccgccgg
cgagcccgtgtggcatgtgcaggcaattgtcagtatttctttttgcttaccccccgcttgtgctccgcatccgatcggggattcaatcccgg
accagtgatgggatctaggtggataccacacccccgtctggagccccggcgccctttccgggaatttgctgggagtatttgtttatggga
ccccaccattgtccagaagatcggcttgctgtttttagttttttttttattgttacaaaactgacctcgccaccctcactccagcatccgggagt
tttccgacttgcatatgcctatattcatgttcgataaggacaatgactttgtggttatgaagcttgaggctgtaagataccccgccgccacca
acatcggcatgtttggtttttaa-3’
Expression primer sequence 1 (SEQ ID NO:45)
   5'-gtcggcgcgccatgtcgctcccgccctcgatcc-3'
Expression primer sequence 2 (SEQ ID NO:46)
   5'-tgtttaattaattaaaaaccaaacatgccgatgtt-3'
*Pyricularia oryzae* Ung (SEQ ID NO:47) 
5'-atgtccaccctcaagcgcaaggccaatggaccgctgtcagcttcagatgccaagaaacccaaacagaacgccagcatcacatcat
tctttgccgcacctaagaccacaccggccagcggcgccaccacatctagcagcccaagttctacagcggcgccagcagcctcagctc
cagcccatccaccggctgtcagtttcaacaagcagaaatgggtggagaagcttaccaacgagcagagggagctgctaaagctggag
attgacactttggacgagagctggcttgcgttgctcaaggacgagattaccacaaaggagtttcttgacctgaaaaggttcctagacagg
gaagcgaatgcgggcaagaaggtgtttccaccaagagaagatgtctactcatggtatgtaaaccggcgagcctcgttaccgtcacaag
cggacatatgcgtcactatgttctgttgtatagaggtattgacatgttccgtttcgttttgccacacacaggtcccgccatacgcccttccac
acagtcaaggtcgtaatacttggccaagatccttaccacaatatcaatcaggcccatgggcttgccttctcagtgcgaccaccgactaag
gcaccaccatctctcaaaaacatgtacatttgcctcaagaacgactaccctgaattcacgccgccgcccaacaactcgggcctgctaac
gccctgggcccagcgcggcgtgctgatgctcaacacatgccttacggtccgggcgcatgaggccaattcgcacgcaaaccggggct
gggagaagtttacgcaaaagatcatcgagctcgtggcgggtcgtcgcagcaggggcgttgtattcatggcttggggtagccccgcag
ctaagagggtgatcaaggtcgaccagaagaagcacttggtgctcaaagcggtgcacccgagcccattgagcgcgtcaaggggattct
ttgactgcggtcactttagaaaagccaacgagtggttggtgcagaggtatggggacggggaggagatcgactggagcctaacggag
ggagtcagcctcttgcccaagccaaaggtcgaggtggtcaaggcggcacccgaggcaaagaaagtcaagaaagatgaggaggagt
ttgactttgacgaaggggaagagcttgaggcgctggaggagttgcgaaagatcgaagacaacaagaagcccgaagttccagcacctt
tgactgcgaagccaggggtcgaggtcaaaggagacgagaataagaatccttctacggctacagcatga-3'
Expression primer sequence 1 (SEQ ID NO:48)
   5'- gtcggcgcgccatgtccaccctcaagcgcaaggcc -3'
Expression primer sequence 2 (SEQ ID NO:49)
   5'- tgtttaattaatcatgctgtagccgtagaaggattc-3'
*Pyricularia oryzae* CDA ox and Ung ox (SEQ ID NO:50) 
5'-atgtccaccctcaagcgcaaggccaatggaccgctgtcagcttcagatgccaagaaacccaaacagaacgccagcatcacatcat
tctttgccgcacctaagaccacaccggccagcggcgccaccacatctagcagcccaagttctacagcggcgccagcagcctcagctc
cagcccatccaccggctgtcagtttcaacaagcagaaatgggtggagaagcttaccaacgagcagagggagctgctaaagctggag
attgacactttggacgagagctggcttgcgttgctcaaggacgagattaccacaaaggagtttcttgacctgaaaaggttcctagacagg
gaagcgaatgcgggcaagaaggtgtttccaccaagagaagatgtctactcatggtatgtaaaccggcgagcctcgttaccgtcacaag
cggacatatgcgtcactatgttctgttgtatagaggtattgacatgttccgtttcgttttgccacacacaggtcccgccatacgcccttccac
acagtcaaggtcgtaatacttggccaagatccttaccacaatatcaatcaggcccatgggcttgccttctcagtgcgaccaccgactaag
gcaccaccatctctcaaaaacatgtacatttgcctcaagaacgactaccctgaattcacgccgccgcccaacaactcgggcctgctaac
gccctgggcccagcgcggcgtgctgatgctcaacacatgccttacggtccgggcgcatgaggccaattcgcacgcaaaccggggct
gggagaagtttacgcaaaagatcatcgagctcgtggcgggtcgtcgcagcaggggcgttgtattcatggcttggggtagccccgcag
 
ctaagagggtgatcaaggtcgaccagaagaagcacttggtgctcaaagcggtgcacccgagcccattgagcgcgtcaaggggattct
ttgactgcggtcactttagaaaagccaacgagtggttggtgcagaggtatggggacggggaggagatcgactggagcctaacggag
ggagtcagcctcttgcccaagccaaaggtcgaggtggtcaaggcggcacccgaggcaaagaaagtcaagaaagatgaggaggagt
ttgactttgacgaaggggaagagcttgaggcgctggaggagttgcgaaagatcgaagacaacaagaagcccgaagttccagcacctt
tgactgcgaagccaggggtcgaggtcaaaggagacgagaataagaatccttctacggctacagcagggcctagcggttccggaatgt
cgctcccgccctcgatccaaaagctgagcagcagcgacgacgcggcggtggatgaagtctgcaagacgtacggcatcagcaggga
gcagttccagcagttgcaacgccgcagcgaggcggcaaagtcgacggcctactgcccctacagcaacttccgcgtcggcgccaccc
tgctttcaatcgacgacaagaactcgtacataagcggcgccaacgtcgagaacgcatcgtacccagtcggcacgtgtgccgagcgcg
tggcccttggcaaggccgtcaccgagggtcaccggcgattcagggccctcgcggtgtccacagacatcactccgccggcgagcccg
tgtggcatgtgcaggcaattgtcagtatttctttttgcttaccccccgcttgtgctccgcatccgatcggggattcaatcccggaccagtgat
gggatctaggtggataccacacccccgtctggagccccggcgccctttccgggaatttgctgggagtatttgtttatgggaccccaccat
tgtccagaagatcggcttgctgtttttagttttttttttattgttacaaaactgacctcgccaccctcactccagcatccgggagttttccgactt
gcatatgcctatattcatgttcgataaggacaatgactttgtggttatgaagcttgaggctgtaagataccccgccgccaccaacatcggc
atgtttggtttttaa-3’
Expression primer sequence 1 (SEQ ID NO:51)
   5'- gtcggcgcgccatgtccaccctcaagcgcaaggcc-3'
Expression primer sequence 2 (SEQ ID NO:52)
   5'- tgtttaattaattaaaaaccaaacatgccgatgtt-3'
*Pyricularia oryzae* mre11 (SEQ ID NO:53) 
5’-atggcgccttcaaccaacaacggtgagctgtaaatgttgccctttggcaatttgatttagtacagaagacgacagttaatatctctgac
acgctctaggtcccgattctatccgcatactcgttgccactgataaccatgttggctacaacgagcgtgatcccgtccgccgggatgata
gttggaggactttcgatgaagtgatgcagatcgccagagagaaggatgtaaggcttgagattaggccacatgctcatattcagctgcta
atgtccatttcccctgatccaggtcgacatggttcttctcggtggtgatctctttcacgataacaagccctcccgaaagtccatgtaccagg
ttattcgcagtcttcggcaaaattgcttggggatgaagccgtgcgaactcgaattcctgtgtgatgctgcagaggttttcgagggtgcgttt
ggtcatgtaaactatgaggatccagatataaatatctcgattcctgtcttctctatccatggaaaccacgacgatcccagcggggacggc
catttctgctcactcgacctcctacaggctgcagggctggtcaactactttggtcgtgtgcccgaggcagacaatattgaggtcaagcca
gttttgttgcaaaaaggccgcaccaagttggcgctttttggcatcagcaatgtacgagacgagcgcatgcaccggacgtttcgtgataat
catgtcaagttctttcggccaaaccaggccaaggaggattggttcaatcttctcacagtccaccagaaccaccacgctcacacagccac
aagctatcttcccgagaactttctgccagaatggatggaccttgtcgtttggggccatgaacacgagtgtctgatcgatccgatcgagaa
cccagagactcggtttcacgtcatgcagccgggatcatcagtagcaacttcgctggtcccaggagaagcaaagacaaagcacgttgc
agttctcactgtcactggaaaacaatttcaggtcgaaaaggtcccgctgaaaacggtacgaccatttgttaccaaggaaattgtgctatcc
caggagcccaagcttaagaagcttgccaaggacaaagataatcgagccaaggttaccgaacaactcatatcaatcgttcaagatatgat
caaggaagctcagggtcagtggttaaaattgcaagcgctagagggcaactcgaccccaggtccagcagaaatgcctctgccacttatc
 
cgcctcaaggtcgagcatagcgcgccagacggcggcgaattcaagcttgaaaatccccaaagattttccaatcgctttgccggcaaag
tggcgaatacaaacgatgtcatctacttttatcgtaagaagtcgggcgtcggagcaagctccagtaagtggagtgcactgccctgtggc
gcttgaacatacactgacagaaaaacaacagaagacaaaaagcccctagcgatccctaatgttatggcagaggatggcaatatagaca
tcttgaaggtggaggaattggtatccgactatctcgccgcgcagtcagtcaagattttaccacagaagcagatgggtgatgctgtcaacc
agtttgtcagcaaggacgacaagcgagcgatggaggacgttgtcattgactccttgactacccaggtcaagggtatgatggcacttgag
acagacgaaaacgatgtcgagaaggaaatggctgcacttttcaagaagcaagcgtcaaagtttgaagcgggtcatgggtcaaagaaa
agacgcatcaacgtaccgcaaccggatgactatgactcggatatcgaaggcccatgggctactaatcctcgctcagtcatagaacttga
cgaagatgaggatgacgaacaagatacagggccggcacccaagtctagtcgtggacgtggcaagggagcctcggctcgacgaga
gagtgatggggacacgttcatggacgattttgaagacgaggctgagccaccgaggagaacagctccgtcacgggcagcccccaag
gcagctgtgccagccaagaaagcgtcggcaaggagcagccgcaagaagggcccctttgaggatagcgatgatgacgacgaagatg
caatggccgttgatgaagaagacgactttgaagaggcccctccacccaaacccaaagcaaagccaactgccccaaaacgggcagtc
gcaacacgtagcacggcggcacgtggaaagcagacgactctcaacttttcacagtctcagcgtacaacccaaaagggtaaggggtca
acgcagaagccactggagatcagcgacgacgagatttcagatgatgacggattcgagccaatgctggcaactatgagcaggaaacgt
tga-3’
Expression primer sequence 1 (SEQ ID NO:54)
   5'-aaaggcgcgccatggcgccttcaaccaacaacggt-3'
Expression primer sequence 2 (SEQ ID NO:55)
   5'-aaattaattaatcaacgtttcctgctcatagttgc-3'
*Pyricularia oryzae* rec8 (SEQ ID NO:56) 
5’-atgttttacagccacgagagtaagccggtttacaagtagctgctcactagttgccctcaacagctcgagctgacagttcaatctgatta
tatcagtcttgacgagccagaagcatggcgttgctacagtctggtaagatgccgctcacctcgtcacctttcactataaggtgtgaatccc
gtaggctaatgcgttcaaattctcagggttcttgctacgctggataggagctgtgccagccgcaaagtatctcgaaaggcaatacagga
agtaaatgtcccaaaggcgtgcgtcaccatcgacgagccgccaggagcacccatcgccctgaggctccaggcaagcctactgtatg
gcgtatctagagtttatcagcagcaatgccactacgttctgggagatgccgaaaaattccgcatcagcatgcgaggtctgatgcgcgtat
tggactctggcaccttggatccaaatgctgggagggcgaagtgagtgtgaaaccagttacatcttccaccaaaagtcgctaattcctggt
acaattgtaactaacgatgccctcagaaaaaagcagttgatgatccaagacgaccctgagtttcacatcagtaacttttttgacctgcctcc
gcttgaccttgatgagctaggtaacttgagaccaactgctttcgaatctcagttcaccggcaagtattctcaactttcaccgttttcttcgcag
caacgatcatcgtctggacgcaactcattggccatggattttggtgcggcttccttctttggaagccagtttggccctgacccgtttggag
gagacagttctgcccagaaacctcatccgggggcagacatgtttgctcaggatgagctacctctgcgcctggaagattttgacgctgga
ttcaccattgatgccgacgggaacatccacgaacatccagagccggagcaacctcaggcagctgttcctatcgccgcccatcatatgct
agatcacccaggtccgcagcaccattcggatgatttcattcctcttatgcttggagatgaagccatccttaaccctggcgatgcgccactg
ccagacgcagatgccctcccagtcagacagcatgagcaggaccctgattatgcgcagccagaagacgatgctgttgtcactgccgctt
cgaggagaaagggacgccgtcggaaggcccttgcgccagatacagccaccgaggtcagcagaaccgaattccgcgactggaccg
 
agaattatgtacagagaatggaggaagagaacaagcgtcacagagcgcgtcgggatcatgtgacaaaggaccaggcagcccagaa
cgcctataacatcatgtttggtcgtggcataggtggggttggtcaggacatgggccttccaggcttccaacatgagctagctgagcacttt
gccggccacggccttcagatggcagtttttggccgtatcgtcggagacgacccgcatggtcgacgcaggtcggcttcgcaagctttcg
gcgacgaagaagattctggatccgaagaaggccggcgtgtccgccctcgatctgacgatggatatgaagcccgtcaaagactctcac
aggagagacagctcgaagacgatggcaatggactcaattttggcgactttgatcctctacccgaggtcggccgcgagataggccacc
cactatcagaccagcatccaccctggcaccgccactccatactacacggatcatcaatgaaaggcagtagcgccaaacctgctgctgg
tcaaggccctcgccaagttcaagaaagcccgctccaacatcgaggcagggctgtccaagagtttgtgcgatttagcgacaacgatggc
ctcggattcgatggcagcgctggcatgggctctgtcgatgaccaccctcctgatcaacctgactttcacttccaggaggaccccgcaaa
tcacaacagctcttctatcagcaccgagggccgcgacttcctttcctacgttcaagcacatgcgcgtgagacaggcaccccgcgtagc
aagggagacgagctttgggttgactttgccggcatattccctccaagctcgactgctcgatttaatgcggcatcagcattctaccagctgc
tgtgcctgtcaaccaaccgcactatcaaggtcgaccaagagggcagagaccgtaacaacatctttccttttgggaaaatcgaggtcggt
gtgtgtattgacgctttacaatccgaacaaggcgaggagagtgattatgatctccccagtattggtgatagggcatatcataatgacggc
aatgacgacaacgagatgggtaccgaggctgatgccctgcaaacgcactaa-3'
Expression primer sequence 1 (SEQ ID NO:57)
   5'- aaaggcgcgccatgttttacagccacgagagtaag-3'
Expression primer sequence 2 (SEQ ID NO:58)
   5'- aaattaattaattagtgcgtttgcagggcatcagc-3'
*Pyricularia oryzae* spo11 (SEQ ID NO:59) 
5’-atgctcaggcactcggagcgtgtccgcaggccttcgaggcgtctgctgttatcctctgttcctatggaatcccaagttcaagcgagg
agtcaaaatgctcgagctagtgacggtcgtcaagaacttaccgggaaatatcaaggttctggtcatccagacgcccaggagagaagcc
cgagcgctgggtggtttgtggaacacagcaataactgtgtacagcaacaggctatctcccatgctgccagcccgccaccttcgaatgc
agacagccagcctgtgactgtgatttcacggattgaagccatcttcgagtcgattgcagccgatatacaacagcgcagggaattgtctat
cccgtattgtcgtgtcagaggtactcgtgacagcaatagccagctaagatttccaggacgcagtgtttctgaggcaaggtcgtttggtaa
gattcctttgctcaaccctcggagacatagtacgtttctgcgggttgtatttaaattgtcctgtttccgtagcccgggtgctatgcgtcctcga
gatttcgcatcaggctcttataaccggcaatcttgtgacgaaaaggtgtgtcattaacatcagacgtaaaacaacaaggtacgcctaggg
atgagttcaggcactaattgctcaccaggaacatattttaccaagccagagatctattccgcgagcaggccgttgttgataggctggtag
atgacgttgctttcacactcggagtcgggcgacattcattgaacattgtcggtaatccagatcaatagaagctacctggtggcaatggcta
acccatcttgaagaccgccgctggcaagggtctaatcataggtccaatttccttcgtcctcaacggtggcgccagggtctgttgcgacaa
cgtgcggactgaggtaatcacccccatgaaactctctggaatggattgaatctggagtggccattatgttgattgtaatgcccctctaggg
aacaattatttgttcaacagaaatactaggccgatttgagtttccgtctgcacgttgggtcttgatcatcgaaaaagaagtgagccgaatac
atttgcagaaaccttggtgcctacaacactgacattcaatgcaaggcaacttttaggactcttgcgtcctcccagttctaccggaactcac
gtgccggacctggtgttatggttacggcaagttaccgtcccagaacttcagctggcaatggcctcatatggagaatgctctggctaacca
tacccttttggttatcatagggaaaagggtatccggacctcagtacgagaaacttggtgtactgtcttcgggaagcacgcccgcggatcc
 
caatctacagccttgtggattatgatcctgacgggatcagaatactgtgcacgtataaatacagcagctctgctttagagcatgagagaca
atcggctgtgagcaacatccaatggatagggattcgcttgtcagatatcaaggagcaccatcttagaccatgcaacagcgtcttgcaag
atgctggctcccccgtcaacactgaaagtacgtgccatcacagaacaattcgccttcaatgaggaagattttccaagtcaaagaagctgt
ggccttccgcacgagggtatataatgctgagactagtagcctcttgctaacttaagtgccttgtccgccatgcagtacactcaggattgga
cgtaggacatgttcggaatcgaccggactttgccatgtcgcgacagaacgctttcgaagcaaacacttcccagctgacaaccagggat
cgtaaacttgcacatgagttggtctcaaagctatctggatcattagaaacgaggggagaacatgacaaatatttgcgcgagcttcgagtc
atgctcatgctcaatgtcaaggccgagatacaagctgttgacaacctcggggatatcaccgagtggctagacaaaaagctttgctccca
gttgacgaatgcatag-3’
Expression primer sequence 1 (SEQ ID NO:60)
   5'- tttGGCGCGCCatgctcaggcactcggagcgtgtc-3'
Expression primer sequence 2 (SEQ ID NO:61)
   5'-cccttaattaactatgcattcgtcaactgggagca-3'
*Pyricularia oryzae* symC (SEQ ID NO:62) 
5’-atggccatcaagggatacacagttttgcgccggaagtggaagtccaggaagcccatttgggccctgatgttagccgagctgatca
ccatgattccactgctcgtcctatttggcatcgcccaacccgataccttccgaacgctcatgtggagaatcggctatgccgagaagctca
actcgaaccccaacatcatcctctacgccctggcaaatcatgagccgattcccaagctgccattcgtttggtcgcaactgtacgtcatcg
cgaggttgccccttccttggattgcttgccgatagaaccttaagctctaacatggaagcctttcacagacttaccgagttcaatgtggccat
ctcagtcatgtcactcttcttcctgctggcgaagatgatcggcttcatcatgaaaatctacttcccggtcattgcggttggctgcaatatcgtt
atgacggcagtctatgccgttagcgtctacgggcaggccggtcccgactaccttgaccctcggtacccgagcccgacgccttggtatct
ccggaggagttgcgacgtagccgaggggtatggcgctggcaaaacctgtatgataattcagggtacatttgctgctactgtcgtcatgct
gtgagtaacgctttcttttgtttttatgttggctacctggacgacttttggcttctaaggttcgataacgactcaggagactgacctgcctgaa
ccatagctgcatttacgtgataaacctcggatggtcaatcttcaacatgatacccacaaaacatgacaaaacacacgatgaggatgagg
atggagaagagtacgcgtctagcgactcgcccaccgaggtcaagacgtgggagatgcagccacagacagctggtccaggcggtgt
accatttacccctaggacgcaggctttccacaccttagacagaaaattgccattcagggcatacgccgaacagaggcagcagtattctt
ga-3’
Expression primer sequence 1 (SEQ ID NO:63)
   5'-aaaggcgcgccatggccatcaagggatacacagtt-3'
Expression primer sequence 2 (SEQ ID NO:64)
   5'-aaattaattaatcaagaatactgctgcctctgttc-3'
*Pyricularia oryzae* symB (SEQ ID NO:65) 
5’-atgctgccatcaacaatactcgtgctcgccagcgctgcgctgagcacagcccacacagtcatcacctacccaggctggcgcggc
gacaaccttgtcaccaacgagacctacccctatggaatgcaatggatctacccgtgcggcggtatcaacttgacgcaaaacaggactta
ctggtccaccaagggcggcgccgttgcactccagcccggttggttccagggtcaccagacggcgacgttgtacgtcaatctcggaatc
 
ggcaacaaggggcccgatggtggaccactcaacatgtcgctgtcgatggtgcctgcctttggcatctctggcccgagcaagaacccct
acccaggcacaatctgtctaccacaagtttcgctgccgcaaggctacacgcctgaggtgggagagaacgccacgatccaagttgtcg
agcttgccgtgcacggtgctgctttgttctcggtaagtaatgctcctataataccccctggcgggactaagtatcactggttgtggcatagc
ctttgactgacacgaagtgcccctggacagtgtgtcgacattacctttgtagaacccgacgacgagcgcctcgaaaaggtgcacgcag
acaactgcttcaacagcaccgacattggcttcactgatatctacacaaccaaggtggccaacacgaacatcacctcgacatcagctgcc
gccgccagcttacggcaacctggaggcctggcatggtatgctggctggattcccatggttggtcttggtgcatgggcattgctgtaa-3’
*Pyricularia oryzae* mak1 (SEQ ID NO:66) 
5’-atgtctcgcgccaatccaccaagcaactcttcggggtctcgcaagatctcattcaacgtaagcgagcaatacgacatccaggatgt
cgtgggcgagggcgcttatggagtcgtttggtaagtacacctctgccggaaccgaacaccgcatcttgcctggacctgctccgcaaca
tccaaagaatggctgtacaatttggtcaggacggtaacctttcaaactttgcgacttaatggctgacatgaccggctgcgcgttttgctctg
ttgtagctctgcgatccataagccttcgggccaaaaggttgccataaagaagatcaccccgttcgatcattctatgttctgcctacgaacc
ctccgcgagatgaagctgctccggtacttcaaccacgagaacatcatttccatcctcgacatccagaagccccgaagctacgagacctt
caacgaggtgtacctgatccaggtccgttgctgctgaacactcctgaaaccgaaacgccgtctactgtacttgccagctaaccgtcatgc
gttcgaccaccgtataggagctcatggagactgacatgcaccgtgtcatccgcacccaagacctatccgacgatcactgccagtacttc
atctaccagactctgcgtgctctcaaggcaatgcactcggcaaacgtgcttcaccgtgatctgaagccctcgaatctccttctcaacgcc
aactgtgatttgaaggtttgcgattttggtctggctcgttctgccgcctcgcaagaggacaactcgggtttcatgacagaatacgtcgctac
gcgttggtaccgtgctcccgagatcatgttgacctttaaggagtacaccaaggctattgacgtgtggtctgttggctgcattctggctgaa
atgctcagcggaaagcctttgttccccggaaaggactgtaagttacaatggcgttcctctgtattgagtgtgctacttgctcccgccccag
agtgtgctgcaccgtagacacgatgttctccccagtactgacacttgatactttgttagaccaccaccagctcacgctgattcttgacgttc
tcggcacacccaccatggaggactactatggtatcaagtcccgccgtgcaagggagtacattcgctcgctgccgttcaagaaaaaggt
tcctttcaggacactcttccccaagacttcggatctcgccttggacctgttggagaagttgcttgcctttaaccccgtcaagcgcatcaccg
tcgaggaagctttgaagcacccttaccttgagccgtaccatgaccccgatgatgaacccaccgcaccgccgattcccgaggagttcttt
gatttcgataagcacaaggataacctgagcaaagagcagctcaagcagttcatctaccaggaaattatgcggtaa-3’
*Pyricularia oryzae* mak2 (SEQ ID NO:67) 
5’-atgtcggatctccagggccgcaagatattcaaggtgttcaaccaggactttatcgtggacgaacgttacactgtcaccaaggaactc
ggtcagggagcctacggtatcgtgtggtacgactttcttctgtcttcatcctccacttttcccgtcgcgcggccgccattctgaaagcccaa
tggcgcggccttggttgcgcctgtactgcttcctaaagacctgactgacacctgacaccttgctgtttcccactttctagcgccgccgttaa
taaccagacgtccgagggtgttgccatcaagaaggtcaccaatgttttcagcaagaaaatcctagccaagcgcgccttgcgcgagatc
aagcttctgcagcatttccgaggccaccgtaacgtcagtttcgcataattacctgcgcttgatgctgccaatcagccggcgcatatcatgc
ctggtatagctggacaagtgttgacacgcattcatccgccttagattacatgtctctatgacatggacataccccgcccagataacttcaa
cgagacctatctctatgaaggtatgcacccgcattacatcaaggataccatagggcaggataccgcagccacagtcttgagccgacaa
gaaagagcccgtgtatatgaatcttgctaacatcttgaacctccatgtagagctgatggagtgtgatctggctgccattatccgatcaggc
cagcccttgaccgatgcgcacttccagtctttcatctaccagattctctgcggcctgaagtacatacactcagccaatgtgctgcaccgtg
 
atttgaaacccggaaacttgctcgtcaacgccgattgcgagctgaagatttgcgactttggtcttgcgcgtggtttctcggtggaccctga
ggagaacgctggttacatgactgaatatgtggctaccaggtggtaccgtgcacctgagattatgttgagcttccaaagctacacgaaag
cgagtacgtatcccctctggcgcgcccctttggttttcaggctaggaagcgcatttgtgtttgcgttggtgtccttgtatccatgactaatatt
ctctacagttgatgtatggtcggttggatgcattttggcagagctgctgggaggcaggcccttcttcaagggtcgcgattacgtcgacca
gctcaaccagatccttcatatactcggcacaccgaacgaggagaccctttcgcgaattggttccccgagggcccaggagtacgtccgg
aacctgccgttcatggccaagaagcctttcccaacactcttcccgaacgccaaccccgatgcgctggaccttctcgatcgcatgctggc
gttcgacccctcatcccgtatctcggttgagcaggctctcgagcacccgtatctccacatctggcacgatgcctctgacgaacccgactg
cccgaccaccttcaacttcgactttgaggtcgtcgaggacgttggtgagatgcgcaagatgatcctggacgaggtctaccgtttccgtca
gcttgtgcgcaccgcccctggtgctggtggccacggtgcaccccacgctccccaggtgccgatccctgcaggtgccggtcaaggtca
atggaaggccgaggacccgcgcccgcaagagtacgtcggtcagatgaatgacctagaggctgagcttgctggcggtttggatcaga
ggaggtga-3'
*Pyricularia oryzae* kar5 (SEQ ID NO:68) 
5’-atgcatcttccaacttcattgggtgccttgcatttcttgatggtgctatggttctcgccgcacagtattgcggcactcggctggggaaa
ccgcgcaaagtcatcgccaccacaaagatcccgcgaggctgcgacgtttcacactgacgcctacgatccgaccttcgtcatccagcct
gctgtacttgtcgacgtttacgccgatgccttgcgacaacttcaacagctagagtcggagcctctatgtcatcgcattgccgctcgacttct
tgtcaccaactgccagctacttgatggcaaggatgaagccactgtactaaccgacagtgggcgccttatccgcgactttgtggaggcct
acgcagcaagtcttgccatctgcgaccttgaacgtggcgatttccacattccttcagcttgttcaccgtttcgagaacatgcacttgcgaaa
gtctccgctcacagagacctttctctacatgtctcctcgtctcaaatacaagcgtgtatctcaggcctgaaatccccggattcagcgtggg
gaacgtacatcagctataggcacaaggcgctgcagttttgtgaagcagctagggctgatcaagagaagagtaggttcacttgtgtgttc
ggaatggaaaggtcctacgactaacatcgacaatctagcccagaacatccttgtgtttcaaaagctcatcaaggtcaccgagcagttggt
cacaggggtcgaaaccgagctccaaaaacgtatgaatgatctcgacgagcgaactaaacaagcaagcgatgccatcgaccgcctca
atcccaaactacatggcttacacgagagctttgggaaggttcagatcatgcacaatgggcttagcgaggccctgagggattcacttggc
gcgacccgatccgttctagaggattcgaccactttgcaacagcttctcaaagtcatgatagacgcggtcctccaacatcactctgaagtg
gcctctgccaatgagcaatcagtcgaaagctacgcggccgtcaccgccgccagccaaaggacatcggaggaaatggttcaggtgct
ctctgcactttcgattgccgcaacatatacggtagagctgaatcagaagctcgtcagttgattgtcttgtctcatcgtttgatatggaagccg
ctaatattgtggtagcaactctctcgtgtcgaagccgctgagctctcaaagcgtcagaaacttatggcaaacgtaagttttctgttcctcagt
cggtatagggcgaaactaacggccaccgccatagggtttggtgcgtctcgagaacataaccgaacagctagttgaaaagtacggcaa
acattccgccaagctcgagtctgtccgcaacataaccaatgacattgtcgagacattgaatgacgcccacatattggctaccgctgcga
agaacacgttcacttttgggtcaagtgggtggcttccgtacgtaacattacctctcggatttgttctcgcgggttcctacggtctctcgccgt
cactggctcggaacatgttgctggctggtcttagggagggcctagctctgctatggtcgtcccgagattcactctgcatatggccgtcca
gtttgacgtctttcattttttctacatcttccaacacaccgctcaatgctacgatttaaccactataagcttgctattttgaaggttcacggcact
tgctgtgacttgcatctatttaccgggcacacattcggcgcaaacactttttacctcatttctcggacacactctggtacttttcacattctcca
cttcggcgttttggcacactcacacatatatggggatcaatggtgagcttggggaaaaatgggaccgcatcaagcagcaaagtggctg
 
ctccagataatagattatggttactgggcacaggtctctacatggggcgcgataatgagccaacactattgccttcaaatgccgaacatg
gactcaccgcctctcgttgaatggactttgcttctcgacaaaaaatctcttttcaataaaacagaaactttggctccatttgcattccgggac
cgtga-3'
Expression primer sequence 1 (SEQ ID NO:69)
   5'-aaaggcgcgcctgggcatttgagaatgcatgggaa-3'
Expression primer sequence 2 (SEQ ID NO:70)
   5'-aaattaattaatcacggtcccggaatgcaaatgga-3'
*Pyricularia oryzae* ham5 (SEQ ID NO:71) 
5'-atgtccgctccgggccagctggctcggaaccgctccgtctcgagtgcccaaaagtctaggggccgagtcaaccaggactaccca
gcgatgcagtccaggacgcaggaccgcgctacggtcatgcgatacttcgagccctgcgccgcgacagcatccatgttcctgtatgcg
caaggcagctcggtggtctgctgtcaccacgacactttgacgatagagcggcgattttcgaggcattcggaggagatacagctcttggc
ggttgatacccagagcgaaacaggggctggacgtttggtggtgagctacgacgcagggcagactgccatagtttgggatctcatgtca
ggtgacgaggtggctcgttttgcttcctatgaacaccttacaaccgctgcatggatgcgtaatggcaacgtcgcttttggtaagtcgtcttg
gtcaatgaatcaagacttgctctataggagcagccgtaatcaaacaattcgtggaagaagaattctgaccgatatgcgcaggcaacacc
caaggaaacattatactatttgagccacagacgtcagagcatatttcttcgagaacactggaccaaatagctgtgacatcactagctccgt
ccgcggattgccggacattcgcaatcgggtaagacgaacaaaggatagatgtgtcattatggatgagctgagctaacaggtatgtcgat
atcaggtatcaaaatgggtctctactcattgcaaatcttcagccgaggttcaccattttgcataatctgacaacctcacgaggcccatcgcc
tattgtaaatctagcctggcatgcctcttcatcgaggcagaaatcagacatgcttgctgtgcaaactaacgatggagaccttcgtatttgga
gcgtggcaaagtcctacaacaacgccgacccggccaaagttgtgagaattctcaaaaagaccgagaactatctgatcggtccaaattg
gatggggtggtcaaagaacggaaggataattcagttctctgaaaagtgagccgcccccatttgcagcaagtatgaacattgatatctacc
aagaaatggagactgatgatgttttgacagcgagacgttatcttgggacgtccggactaagcacgttacccaggacagcattccatcgtt
ggaacacgtccgaggattagctgtttatggcccaggcgccagtctttttacgttgggtgccaacaataccgtacaacagtttgatctcaac
gcacctgcgatgatggtcgccaatgttcagcacccagctaatctgctgccaccatctccgcctgtgtcaatcgaggagcaagaaaagg
cagcggccgcgagtaactcggagtcggagatatctatttcgatcgagccaatgacgtccgagagcgaagaggaaaggatgtcaccg
ctcgcccgcatgattcattctagggacactgcctcgagcggtccggggatcttcaggcctgatagctcacagtcaagccacagtggca
cctccgagatttcgtcgaccagctctcaaacgcctgggaggtaccaaccatcagcccattcgcgaggccttacggaaaacacgtatata
tctgcagggtcatcaatgttgtcatcggcagcaccacattcttctcggcatcgtgaccgagactcgtactccattggaagcctcagctctg
tgtccatggcttcatctagcggccgtccgtcaaaacatcgcccctcacgccttcgaaatgagattccgcgcagcccagaagactcgag
ggtcgatgatctcttcaaattcactcgaagtcgcctcagcgatatcccttacaaaatgccagccatggcagatgcctcgaggctcaccaa
tgacgatcttcgacgacagatgttgagcacgattttcggctggaatcgcgaggtagacgacttgattcgagatgaattaagcaggcacc
ccaactcctcagctaaccgcatcctgctgtcaaaatggctcggcgacatcgagccggacattatcgcaaccagttcagccaacatgac
ctcttcggattggatgcttctggctctgagcggaatcgggggacaggcatctcagcacaagcttgggcgtgcttacgttcaaagacttctt
gagaatggtgacgtacatgcggctgccacaatcatgatcggaatgggcgaccagattgatgcgattgagatttacgtgtcgcacaaac
 
gctacatggaggctctactcctgacctgcctcttcttcccgtcggtctgggaacgccaagcacagattctcaagaagtggggagattgg
gctgttcagcacggccagcaacaattggccattcggtggcaagttttctccttcgtatttggcttaaccctaaggctatttttatattccccctt
tgtaaagctactgactgctaacacctgatgttctacttagccttgcctgcataggcaaagaatcttcagagccgtggacttcgccctctgca
gcacaactcagtttcggtagcctgaatgccataaactctagcatccctgaggtgctcagtcctccattgtcaccgccaggcatgaacagg
ggaccgcagcgcagtatcgcaaaggcttcagcgctgaaactcatcacttcctttggagaccaaggcgcaaaggccaagtactttgcag
gcgacgatggacagacgcctattgccgcaggcgtgacgcctatcgccgagtctgcaatctcccccggcgcctttgaccctgccaccg
ctttcatccgtccctcccaacgaagtgccttccccacgcccagctccgcgcgtccttcgaaccaaagctttggtcgacatcgcctgcctt
cgatcggagaggcgacaggaaagggcgagcagcgtcgacgcacgcccggcccatcgtctcctgtactagaccgtgacggcaagg
ggcatttgagaggaccgtcatacgattactccaacaacaacttgaacgaggccgaggcgatcggccgtgcagcaactgcaagcccaa
tgatgatgcgggacagccaccgcaggagagagccgccgcctccatcgccctcgcctgcttcgatggccatgctcatggatcatcagg
gtgggcgcacgagccgcaacgggccgcgtaatcgcattcctcaaggaattgacttgcaactgcagcccattgagccaccaaatcaga
gcagtgacgtgacttctcccgagcagtctgtggcctcttcgactagatttcactggccaagccgccggcggggacccggctccgtagc
cagctcagtcacctctgcaacttccagcgtagcaaaggcacatcgtgcccgaggatatggccaccctggtaaaactctcgatgattatat
tcacagcatggacaacacggctggcaggcgtgcgaccagccgtggacgcggacgggactcgagcaagaccaggaagtccaagtc
tcgcgaagcatctcttgaccatgatcagcgtgggcgtgagacttcgagaggatacactccacgagctggcaagcgatctcctcgatca
cctgtccccatgtctcctgaggaactaatcaatctcagcaccccaaaaacgaatgatgccagaagagggggcgatggatcagtcattg
agattgccacagatcaacccagcaccgtccgaaaattaagcagcagtcagattcggcatgcgtcaaggactcgagcaaacggtggta
gccggccttccagccgtggacggcgaacggccagtcccgaaaggatcggtcgcgagcgcagcagtggcaggaatgcatcacctcc
atctccagtaccaatgtcggccatcccaagagatttctctggctcagaggaggagaatgactatatgagggcgattgaggcaaaggaa
aggttccgcaagaagaacactcgtagcagtagccggggtttccgtaacgcctcgccggggtcggcttcccaccggctgatgagcgac
aggagaccttccaaggagtctgcaggctttggtgaggctgaggatgccgtcagctcgcttcgcaggatcaaggacgagagacagctc
aagaaagaggcagcagctcgagagcttgaggaaagacgcaagtcactggcgcgtagacctgctgcaccgccgatcccacacccgg
atgagctgtcacctattgttactcgaggcccggccttcttcgagtcggccttgcccagcacggcctttgtgccacccagagatctgccca
cacgcagtcgcacggtcgagccgggagagtcaaggagcatgtatgccaaccgtcaaccccttatcgggcttcctgccacacccaag
gcgatgcgcttgatcctggaaccagagactggaggtgtgccgggagtccctgaaattcctgtcacctttgcccagagagcctccccaa
cacagtccaacgcatctccaaggcattcccccgagaaaagtgaggcggtggccagagcggattccgagcagaccaaggcgcccca
agagaatcttcttactttactgccctccaccgtctacagccctcccgtgcggccaccaatttctcgttgcatgtcagcgccacccgaggag
ccgctcgcccccaagactttcaagccttctcagcaatccagctctcgccaggccaatgaccgtaagatgagcacaccggacgtgtcttc
ggaccttcgccaggcgggcaacatgaggacaatagacgacatgctggcaaacaacacccaaaggccgacgcatgagtcccaagg
gatgccacctcctcctccaccccctccagtgctcaaagaactgcagcatcttgccatacccccaccgccgcctcctgcgccactttcatt
cagccaaaagtcgcagcaacctcaagtctatggagggcgtaccgctggaatgatcgagatagtgatggacaatgatgaccagatccct
cctccaccccctcccccgccgccaatgaccatgtcggcgccgccggttcctgtggctatcccagtaagcgaggccaccgttcctatcct
ctcgccaccggcaccgccaacgcctaagtccaaccaccagcggggtcgcagcagcatcgacaacagcatctcgtcgcgcatctctc
 
gtgtcaccgatcgcgttcgatccgccagccgtagccggaccaactcttcgatgctctcgcgcaagtcgcccgagcttgcgaaccaaac
ctccccatacgagagcattgggccgcccgtcagcttctctgcccgcgctgtggctcaggtccagcagcagcagcgcactcagactgg
tcttcagcccagtgagatgttttga-3’
Expression primer sequence 1 (SEQ ID NO:72)
   5'-aaaggcgcgccatgtccgctccgggccagctggct-3'
Expression primer sequence 2 (SEQ ID NO:73)
   5'-aaattaattaatcaaaacatctcactgggctgaag-3'
*Pyricularia oryzae* ste11 (SEQ ID NO:74) 
5'-atggccatgttggcttcaaagtcgccttttccggcccctctggccacttcaggctcaggaatgatggctgggacagcacaagcatca
taccctccctcgagacgagcaccagctgttccgcctgccagccagtctttcagtccgaccgagtccgagttctccgacagcgatgatca
tgattcaccaaagcactgggacgaagacaaggtctgtgaatacctgaggagcgttcgctgtggcgagtacgagaagctcttcaggaa
gaaccatatcaatggcgagaacctccttgagatggacaaggatgtcctcaaggagatgggcattgagaaggttggcgaccgggtacg
cttgttcctcagcattaagaagctgaggacaaaggccatcgccggccagaagaagaggaatagagtgagtcgggggaatgcttccttt
gcggctcgaaacgcaatggctgactttagttaccgcttcaccaggattcctttgccggacacgagagcatgtacactccagtatctgaat
ctcccagcaagccctttcactctagcagccgtgtcatgcccaatccttcagtgaacaagagatactcccgtcagattgacttgagcggca
tggcatatgatccctccaggccgaccacttcctccaggccaacatcgccgcttcccagtgccgatttccggactgcgcgcacacgaaa
cccatatgttggacagcaacccactcctactgggtcgctgagaggacttggatcaccaccagatagccaggcaaactcacgtccggta
ttaacccatacccggaccgattccggtatggacggatcgctgatggctgccttaccacagaaccaagatgtcattcgtgtcatctctaca
gggggcgtcaccaaggttgtcaagattgccgactgcaacacgtgtgaggaggtgatgcgggtaacattgcgcaaattcgcgctgcgg
gaggaccacgagaggaattattgcttctgggttctttctggacttgacccagatccgaagcagtgccgtagactgggagacaccgagct
ttggcgcgtcattaaagaccagaagcgcccggagcgcaaccgacttattcttcgtcgggtaccggctggcgagccaggacagtctga
gcttgagagggccgccgcgattgccatggaggaggctcaacaatcgcactcccgcgccatggacaacgttggggcgcgtagccag
atcaaggtgcaaaaggttctcggagagaattgggataacctacagcccccgctctctcccgtcttgtaccaggatcgcgagagaaacg
tttacaatgctgctcgtgacctagagagacccgaacctctggattccggccggctgcagcctaggagaaaggtcttgaggtcttttggc
ggtctcaggcctccgagtgaactcattgcttccgacctgaccacttacttccccgaccacccgcgggaggacattgatcggactgcga
gactgtctatgcgtaggtctgcacgccttagcaaggttaacagccgccttagcgttgccagcagttttagcatggcctcgagcattcagg
atgctcctcccatccctaccatcgccgacagctggctacagtcgacacccttacccaaggctcgcccccgagacctgcagagtaggct
gcagcatggctaccgtgattcggtggcgtcgtctatgctagacacgcttcaggaggaagggtctcctatagaaccgaaccgcaagtcc
tatgtttcattcgccgacagcggctcagattctgctgccgtgagtgtcacggaccccgacggaaacattgtccgccacagctatttcgac
gagggtagcaccattggttctggctctggctccggctcgttcggcgatgtcagcaaagctctgaacgaggatggagaggacgccgac
gaagatctgcagagctttctctctggcgagtcttgggatgacagcaaatggatgaagggagcactcatcggacagggatcctttggatg
tgtctacctcgctctccatgctattacaggcgagctcctcgccgtcaaacaagtcgaggccccatcgcctggtgccaacagccagaac
gacgccaggaagaagagcatgattgaggcccttaagcgtgagatcagcttgcttagggatcttcgccaccccaatattgtgcagtacct
 
cggctgtggctcttctgctgaatacctcaacatttttctcgagtatgtccctggtggctcggtccagaccatgctcaactcctatggcgcatt
gccagagcctctggtccgcagttttgtgagacaaattctgaacggtttgtcgtatctccacgaacgtgagatcatccaccgagacatcaa
gggtgccaacatccttgtggataacaagggcaccatcaagatctcggactttggtatttcaaagaagatcgaggccaccaacctcctca
acggcgccaacaacaacaaacaccggccctcgctgcagggttcggtgttttggatggcccccgaggttgtcaagcagacaagttatac
ccgcaaggccgacatctggtcacttggctgcctggtcgtcgaaatgatgacgggtacacacccattcccggactgcacacagctgcag
gccatcttcaagatcggtggaggcaaggcaacgccgaccattcccgaggatgcgagcacagaggccaaggctttcctggcccagac
ttttgagatggatcataacaagcgccccagcgcagatgacctcatgctcagccccttcctgactcctatcacgtaa-3’
Expression primer sequence 1 (SEQ ID NO:75)
   5'-aaaggcgcgccatggccatgttggcttcaaagtcg-3'
Expression primer sequence 2 (SEQ ID NO:76)
   5'-aaattaattaattacgtgataggagtcaggaaggg-3'
*Pyricularia oryzae* whi2 (SEQ ID NO:77) 
5'-atggctgccgcaggaggagcgtcgagtattattactcaggtgcaacagggtggaccgccaatcaactcgatcggaggtatgtgag
actgcggctcggagtaacgggatctggctttctaatgtgtgcatgaactaactgggtcatagatgtcggcgctgatgagacgatcacgat
ggaccttcggggcacgaggttctccctctcccgtgatgaactgttgacattgcccgaattcgtcctgctttcgttgtttcctaatggcttgttc
ccagaaggccacatgggcgggtttgctgacggtgatgccgtccaagtcgatgtacgtggagactgggtttctatccttcctatcttttaatt
ccgatgcctctagttcacctactaacttgattacgaacagtacgacccggcatctctgcagtacatgctggattttttccgtaacgtggcac
agaccatacctgcagactctgaggtagacaacgatatggtgccggtcgagcctatgggtagcagcagggacgactcatccaagaga
gcaggaatcattgtgctccgtgaggacctagatttctatgccatcccgccacggcctgatatcaagcagccagacatgatcgaggtcaa
gcgggccgcggccagggcgctgctcaagcagaacggtatcttcagtggccttaagcggagcgatgagcctggtacgaccgaagcg
catctgatcgagatgttgactgctgggtaggtccccctttggttttaggacatatgagtggactgttttgtgtgctaacaggctggtgtttga
cttccagtggctttgatcacgatgacaaatggggccacagagccggagagcccaataaggcagtcatttgcagtctcgcgctggcacg
gctacgcagcgacatccgtggtaacgaaatgggtagcaatgcggtcggcatggcacaaaagttgttgttgttctggcgcaagccagca
cgcaggtgctggtgggagggtgtagagcttgacaacgtggagggcgtcgaagggaagctcaaggtttggattcgtcgggtgtggac
gcttgagatgagcgtcatcggcctgcgctaa-3'
Expression primer sequence 1 (SEQ ID NO:78)
   5'- aaaggcgcgccatggctgccgcaggaggagcgtcg-3'
Expression primer sequence 2 (SEQ ID NO:79)
   5'- aaattaattaattagcgcaggccgatgacgctcat-3'
*Pyricularia oryzae* so (SEQ ID NO:80) 
5’-atggagccaaatagatcaaggggcttttcggatgaagagctcttcatgagtcgcccggtcgacgactgtggcgacagcccaaccg
tcgagccccttcgcatcttcaacaagagtcctgccgcctcggatcaggcccgtttcaagtacccgcgacccccggagccggcctcatc
ggcttcgccctcccacaagccaacgttcccgctgccccctggcgcatcgagctctgcggcgcccctcccataccccgacgatgagga
 
tctgcgcccttcgaagccgctccaatccaggctgccttacccagacgatgatgttggtcggaggccttcgcccgctggaaggatacag
accagcagtcctgcaagctctggaggaagctacaatgacacatctccgcgcctgagcctcagccccactggcgaaaagagaccagg
tcttgctgaaaggaggggcaccgcgcccagtgcgctgtcgccaacaagtccagatggggacaaggataatctgttcgcgaagcctat
caagccgcccaagcagcaacagccacaaccgcaggagccgcagcctaggatctcaaacagctaccagagcaaaccatactaccct
ccgcctggaggcagctccgcaaacaatgctcagcggccgactgtggctacaaacacgctcaagatgcctcaggaaagcagcgtgaa
ccgtttcaactcgaatgcctccacgtcaaccacccgcgccagtcgcggctcacctccgcctccagaaaccccgatagtcgaaccgggt
gtgattcctggtggtggcatagaggctcgctatgctgcctcgggcatatctgggaccgcgaccctcacggccctccaagcgcaacag
gcgcagagcgccgcggcctcgcagcgacttgcccagtatggtggacagcccccggcacaacgcccgtggacgccgacagagact
ccggaccaacagccccatgggcccccgacggtttaccagggctcaaatccagtgggaagcccgtctcagtcgtccacggcacagaa
cggacaatcccagcagccaagtttgcaagttagcgttctggagcaggatttccagcgcatgcaagcttcaacccctccccctgcatact
ctagcgtcagtggaggtggtaccgggtatccaaacgagaaacagcgccctgcgttgcagcaacagtccagttccagctcgcaacctg
ggcaagcgcctgtcgctgcaagttctgcggccgccactacatcggcttcagttgctggatccagctcttccggcgcagttcagactccc
gttgctcaaggcccggagaagaagccgcaacagcagcagattccaaatcacctggcttcgccggctctacagcatccgggtcatcct
gcctttgccaatgaccccaggccaggaagctcccagaatggccaaccgaccatcacacacacgccatcggttttacattctcagggcc
cctcgtcgccacccccactgccagaaggatggatctcccatctcgaccagaactctgggcagtactactatatccatcttgccactcagg
ctacgcagtgggagttccccaagggtccaaatccgatcaaccacgaggtagccccattgtcaccaacagcttcgacttatggcaactct
cttggttctcctttcctgagtggtaaacagggaatggcatcgcccggcctgtatcctccccacactcctggctacgctgagagtattatga
gcgtggcggcctcggctgcacccagtgctgctggcttcactggacctccccccagctccggcgtggagatcttcaaggttgcccctac
aaatggtgtttactttggcccctacttgcggtacgtcaacatggacgtggagaggggtgtgtggttggggagcataatgattgtcacgga
cgctcctcagccgcccacgatacacattcatcgcagtgtagacttgtcaccgaacccgcggcagctcatgccacatgtgattcataccc
atcaacgctggagcttttacaagtacgacttggatctgcaaatgggcgagcaaactgagaaatggacttatgcggtcacgtcccacatg
ggatgtacgcgttacgagttcgtcgttgcggggcgcagtgaaaccggctggcgcttcgtggcgcactcgggtaacgattttgcctcttc
aacttcgcaaggcgacagggcaaaactcggtggcgtcggcttcatgtggaaggacgtgctgcaaaggaatctggagtgcggcggttt
tcacgtacagctggggctcggtgatcaaatatacggtgatcggctctggaaggaggtgcctctgctaaagcaatggctcgccatccaa
ggaagggagaatcgcaagaccgtgcaatggactgcgcggcatgaagaagacgtcactcatgcctacttccactattacacgtcccact
ttgaccagccattcttgagagaggcgtttgctcagatccctcacatcttacaactcgacgatcacgatatgtaagtctattttgtcttgaacc
ccctatcgtataagtttgattccgctgctaacacgactccctcgtcaccaacagttttgatggcttcgggtcttatcctgactacatgcaatct
tcacaaattttcaagaacatcgggcgtgtggccattgaaatgtacttgttgtttcaacaccatacgactgttgaaatattgcgcaatgttaact
cggacattgatctgttcactataacgggctctgggtggcactttgtcaagtacttgggcccggcaatggtcgtggtgggtgttgatatgag
atcagagcggacccaaacgagagtcttggccggtccaacgtatcaaggcatctttccaaaggtcgccatgttaccacccagtgtccag
cactgcatctttatgctatccgttccgcctatctatcccaggctcgacacggtggagagcttggcaaacacattcaacacgggcaagaag
gcggtcaactcgacatacaacctgctcggcaaagtgaccagctcagtggcgggtgtagtcggtggtaaggaggtggtgcagcacgg
cttcactcacgtcaagaaggcggtcggaaaaactggtctcatgggcaatgttctcaaccagtttggagagtttgacattggtgaggaact
 
caaggacctgtggacgcatgagacgaaagacttggagcggacatacctgatccggactctgcaaggaatttcccagcagaagggaat
aaggatgagttttctttcaggaggtgagtttctttccatgtcttgttcctggaaacccctgccctattaaatatccagtccttctgggcgcggc
ataagttgcaagcccaaaataaaaaggtgactaagctgaccatgatatgaacagatgtcaattgctgcggcgccgggttggttcacgac
ccgtctcaccctagcgaccacaagaccatgtaccaaattatcacctccccaatcgtggcggcaccatcgggtaactacatcctcaagat
gcttcacaactccaataagatgctttatgtgcctaccaacggacacaaatccaatggagaggtgtcggacaccaaggaggacatgatg
gagatcttccaggtcgacgcgaatgggtcccaacgcgagctcaagaagcttatgggacgtcgcaactatgttgctgttgtggcgtacga
ccccgaagtcatcaacgccgcggcaggatcgcagtacgccgcaagcatcatgaatggcggccaacctcagggccaggggctgcag
aagctcagtcttgccgtcgactttgtggttcagggtgacggatcattcaatacgcccacgaccaagtacggacctgtcattatcccacact
tggaatatgggcactga-3’
Expression primer sequence 1 (SEQ ID NO:81)
   5'-catacacaaccgtcggcgcgccatggagccaaata-3'
Expression primer sequence 2 (SEQ ID NO:82)
   5'-ggattgattgtttaattaatcagtgcccatattcc-3'
*Pyricularia oryzae* pro1 (SEQ ID NO:83) 
5'-atgtcaacaatatcccccaaccttcaggccaacctcgcgacagcatggccagtaccaaaaatggctgtcgcatcagcaacaaaga
caaagccggccaaggccgcggccgccactgccaatggcacttccaacaaaaagagccagatgcaccgccgttcgagaacaggttg
gtaaccctcctagcgtgccttaaaatctctcacacaccggacttagaccagactatacttttttttttttttttgcgattcacgctatgaacctct
ataaactaactgtcaaccacccccacttacaggctgctacacctgcagattaaggaggaaaaagtgtgatgagggtcaacatgtttgctc
agcatgcaagcacctcggtctcgtatgcgaatacaagcgcccgcattggtggagcaacaacgaggccaggaggagacacaaggat
gatatcaagacaatcatcaagaagaagaagctcgccgagaagggtgcccacgctgtccaaaccggcatgagctcacccccgggcct
gtctcactccatcccgtcgctgccaacctcggccaccttctcagaccctctcgacaggacacgatccgcctccatcgactcgcactttag
cttcaacttcaacagcccgccaacgacatgctcagaatatggtgcattcactcctcagctacaccccgacttcatgttcaccggaccatat
gcttcgccctacgagatcgacgtcaagactgagaggcaaatgtttgttaatgatgtgcctaccctcagggaatcaacaacctcgaccttt
agcacctgctacaccccgccgccaccgggcacaaccctgccttcgttccctctggaaggtgaatggactgaacaggtgtgctcggaa
cggcgggagtccttcaccgaggagactctgaatgtcaacttctttgattttgcccatggccgctcgatgcagtcaaggcaagttgccatt
gagcttgacgagggtgaccaacgcctgctggaccattttgtccgacatgtcctgccgactatcttccctatcctggagtccaaccagcac
ggctcggtcagctcagacctgatcctgccggcgctccagagcaacaagggttatcttcactgctgcttgagcatcgccgctcaacacct
caaggctgcaaccggctctcagagcgaggacatggatgccgacatcatgcgacaccgctacgcgaccatctctgcgctttgcgacgc
tctgaaccgcgacgagaaccatcaacagattctcgaggccgcgcttggcttgatcttcttccagtgcggcgtcgggcgcttcgacgatg
cccttccagacattgcgtggcatcaacacttccaggccgccatctctcttgtgcagaagcttgaccttccccgcattgtctcagaccccaa
cgcagaggctgcaccaacaccattcaacatgactctcactgcttggatcgacatcctcggtgctacaatgcagggccgcgcacccacc
ttcgcccacacctaccgtgagaagcatttgtctgcgaccaactcgtctctggggcttcgcgagctcatgggatgcgaagaccgcgttat
gtacctcatctccgagattgcctgtctcgaggcactcaggaatgacggaatggacgacatcactctttgccaacacgttcatgttcttggt
 
gaccaaattggtctcaccgaggttggcgagactgcccctaagctgccgttcaactctaatggttcgctgagcccaaagcagttgagcaa
aaacatcacggcggcattcagactcgcggcgcgcatctacctgtgcagccttgtgcccggcttcaaccctacacaggctagctgcatc
ggcctggtcgaaaagctgaccaacgttctccagcacatcccctcaggccaccaaggatttgaccgctctcttgtgtgggtttacctggtt
ggcgggtccgtcagccagcccggctcgtctttccgtggcttcttcgaggaccgcgtggcacaactcggtgaggcagccaactttggct
cctttggccgcgtcagctcgctgctgcgagagacgtggactgaggccgaccagctcgtggccatgcagtctcaggcttgctcccctgc
tgcatcggagacagggagcgtgatccccacacactacatccactggagagatgtcatgcagacaaagggctgggattaccttctcgta
taa-3’
Expression primer sequence 1 (SEQ ID NO:84)
   5'- tttggcgcgccatgtcaacaatatcccccaacctt-3'
Expression primer sequence 2 (SEQ ID NO:85)
   5'-aaattaattattatacgagaaggtaatcccagccc-3'
*Pyricularia oryzae* pmk1 (SEQ ID NO:86) 
5’-atgtctcgcgccaatccaccaagcaactcttcggggtctcgcaagatctcattcaacgtaagcgagcaatacgacatccaggatgt
cgtgggcgagggcgcttatggagtcgtttggtaagtacacctctgccggaaccgaacaccgcatcttgcctggacctgctccgcaaca
tccaaagaatggctgtacaatttggtcaggacggtaacctttcaaactttgcgacttaatggctgacatgaccggctgcgcgttttgctctg
ttgtagctctgcgatccataagccttcgggccaaaaggttgccataaagaagatcaccccgttcgatcattctatgttctgcctacgaacc
ctccgcgagatgaagctgctccggtacttcaaccacgagaacatcatttccatcctcgacatccagaagccccgaagctacgagacctt
caacgaggtgtacctgatccaggtccgttgctgctgaacactcctgaaaccgaaacgccgtctactgtacttgccagctaaccgtcatgc
gttcgaccaccgtataggagctcatggagactgacatgcaccgtgtcatccgcacccaagacctatccgacgatcactgccagtacttc
atctaccagactctgcgtgctctcaaggcaatgcactcggcaaacgtgcttcaccgtgatctgaagccctcgaatctccttctcaacgcc
aactgtgatttgaaggtttgcgattttggtctggctcgttctgccgcctcgcaagaggacaactcgggtttcatgacagaatacgtcgctac
gcgttggtaccgtgctcccgagatcatgttgacctttaaggagtacaccaaggctattgacgtgtggtctgttggctgcattctggctgaa
atgctcagcggaaagcctttgttccccggaaaggactgtaagttacaatggcgttcctctgtattgagtgtgctacttgctcccgccccag
agtgtgctgcaccgtagacacgatgttctccccagtactgacacttgatactttgttagaccaccaccagctcacgctgattcttgacgttc
tcggcacacccaccatggaggactactatggtatcaagtcccgccgtgcaagggagtacattcgctcgctgccgttcaagaaaaaggt
tcctttcaggacactcttccccaagacttcggatctcgccttggacctgttggagaagttgcttgcctttaaccccgtcaagcgcatcaccg
tcgaggaagctttgaagcacccttaccttgagccgtaccatgaccccgatgatgaacccaccgcaccgccgattcccgaggagttcttt
gatttcgataagcacaaggataacctgagcaaagagcagctcaagcagttcatctaccaggaaattatgcggtaa-3’
Expression primer sequence 1 (SEQ ID NO:87)
   5'-aaaggcgcgccatgtctcgcgccaatccaccaagc-3'
Expression primer sequence 2 (SEQ ID NO:88)
   5'-aaattaattaattaccgcataatttcctggtagat-3'
*Pyricularia grisea* MAT1-1 region (SEQ ID NO:89) 
5’-catctgccaagctcagctttcacttacaccgagcatgggagagacgcaagggatgaagtccattccaaaagaaacccgccaaatc
gcttcccataaagctttctttgtctcaaagccccaccaatctcgcttggacctccataccaacttttgaaatctttcctctttcatactttcatatc
accctttcgtgcacacatttagcattcaatctgcttgaagctgcaatacaacggcatttcaaaacgacgcgatggcgcctccctcaagtgc
ttccaatggagacaagattcggcaaatgattgccagtctctcgcctgacgacatcgccaggctgattccccaggagactctgacttctct
gctcagagcaaatgacgagaaagagcgcctgcgggagctgcccgttagcccgagagcggtagcagcggcgagcaaaaacaagaa
gaaagtcaatggatttatggcatttcgatgtgagtacagactgataatagagaaatcgaagcttgtgctgacagcagtatagcctactacg
ctggcatctttcaggatagaccgcagaaagaacgttcgcccttcatcactctactctggcagaaggaaacgctcaagtcaagatggact
ctcatggccaatgttttctctcgcattagggactttgcaggcaccacaagaggtcgtatggcaatgagtggattcctgcgagttgcctgcc
cgcttctgggcatcaccaaaccctgcgactacctcagacgctacaattgggaactcgagtttgttgcagacgcgtcagctccgtatgatg
ctgctatgaaatacgagatttcccagtcacaaatccctcacattgtcgacgaattcgaggtccctactacggagattgagttgcttcgcgc
ctgcgttcagggtggctttccatttgagaacagtgcccagcttctaagagacatggaagacagctctgtcaccgttatgaccagaactgc
gcctatcatggcaccctcgcatgcttcgcaggcctcgcatggccaacataaccaccatttcatcaacacactcatcaacgacccggatg
ctgcgatatctgcgcttttgccccaagatgaagacattggcagtctgatggttgacatgaatatcatccactcactcgagacagacagctc
aacaacatcatcggcacgcaactcagtttctcctctggagcagcacttgttctttcacgaagacgtctccatcgatccttcaacaatggttt
ctttccctggcgaaggtcatggtcacccggaaacacaatacagctaccccaatccaacgctaggtctctggtaaatcagaccatctcag
gagctcattttagtctagtttttattgagagacaggtcattagttgtcacataataattctcgcttgcatctggtcattttattaaaggcaggctc
atcaaattagcgggtgttttttcttcaaatatcaacgacgagacaaattcaaccattccagaacaaaagcttcttaattcagtcggagcaatc
attctgtgagaatgctggtgaataagtattttagcctactgataggcacggtacgtccactctcgatgagtctgcatacccgtcacaatccc
caacgaccaagccaagtcccctgaagcccgcagaaagtgagagagaaaaacaaaggtaagagttcccttatggtcgtaagcaggtat
ctcctaagtatcggaggttagatgagcttgaccagagaatatggatacaagttgaaacttaccccaagtgtacctaaggcaagaccatg
catttgatagtagttgaaggtatgagctacacagttctgttaaatattagtaatgttttagtttctcaaaatagaagagtcaacaagcctacca
ctggaggatttagaggtagccgcgcgcgatcatttgcaatctttatcttgggtcttctcaaagttagcagtgtaaatacagggacatgttag
agtgcatacctgctcatcttcatagctcaagcgtatgacgctttcacgcagtgtagcagcttgctgacgtgcggtagtgtagcaccccatt
gtagaacgtatcaaggagttggcgcagtgagttgttatgaaagtaaaatcgcgcagcttttcttgatcgaagaaaggcaggtctaggac
ccgatgaaacaggccccatcggctgcccggcattgttggaagagaggggtgctcgataatatcaagccattgcccgtagtccttagga
tgactgtgctctctgagaacaaggcaaccatcttggaccctgagaaggccaatagaagcgctgacagactgagcaactggctcatact
ggagtgtccgcaccctcattgccagaagggtcaccacggcggccagaagacaggtattgcctgagttaaccttggttcgctgagataa
atcgaagcaaagcgaaatagcatagctgagctttcgggcgtgatctgaatcacgtatggcgcatcgggctcggtgtaaagcaccctgc
aggctctgctctgtccacttctttggaagaggctcagagccgtctttgtaagccttcagtgtgtctaataatgcttgcagggtaaaaaactct
ttggttgcttgcaaagcagagacaatggcttgatggcactcggtgttttggttcatgctgctatcgcctaagagttccgcgacagaaccga
gaagggtcttgcatgcagatacaaacgggcgcgctcgatagacagcaatatcctctggctctggttttccattcttgtagaagttgaagg
gaggaatctttatgtatagataatctgatgcagttgacattacgccgttgacgcgacagtcatggatttccttggctatttcgacaggtctcc
gggaagctgctggagtctaagagcagtaagattgggtatggcaaatcgcttcagcagatgagcctcaggtgagaaacgaaatcaaca
 
atcgacaacctggcccgtgggtctgaacaatagtaagcccacaatcaatacacaataccaaactgccatctgctccactccacgctcca
catattggtattgttcttcaacactccacctcacaatagcaacgatactactattatgccctccaatctcgaaaaactataaatacttcccttct
ctccctcctctcgacagatcttcaactctctctctctctctctctctctctctctctctctctctctctctctctctctctctctctctctctct
ctctctctcttccacatcaaacagctcttcccaaaaaacttcacttacagcaaatatcacaaactggtatctcatcgtctcacgcaatggact
acgagaacagcatagtggcgagccagatgcctgcagagaggatatctgtcagcgtctcgatacctcaacaaacagttgatttcgaggt
gccggccctctggtcgctaggcaaagtttgcggcatcgcttcggagctcaggtaagccgacccagagtcctcacaccagagcgacag
ctcttcagcatctaagcgatcacaggctaacacttttggcgcctcaattagcaagaacatggcctgcatggtttggatctgggtcgtctgc
gttcctcagaagcggtaccgcatcacgacagctcgctggaactccagtgggctgctggttggccgcgttgatcgtgagttttctactttct
tcaccttttccttccctcccagatctgagcagagactgacaagcgacacaaaacctccccacagctcctcctcaatctgccaccacagcg
tcctcacagtcatcgtcattcgacggcagtgaggaagacacaaacacaaacatcaacgccaatggtatcgatgagaacctgctgccgc
ctgctctccgcggccgcactggaccttccaagcctccaaaccgctggattctctaccgtgccgccaagtcagctgagctgcgggcaga
caacccatccatgaacgcgagcgaaatctgtaagttcttcgctcttttcactctttttctccgctaccacttgtacctaccttttccccatctcc
catactctttgaaaagtctccagtcccacctctcaagctgctgttttgacaagataaaagactaaccaacatctctctcctcacacagctca
agttgcatccctgatgtggcaggcagagtccgccgcgacaaaggcagagtgggaggagcgcgcagccgaagcccgcgaggagc
acatgcgaaacactccagagtatgcggtggcggccggtctgcgccagcgcgccatcgccgagaggagaaggcagaggcagcaga
agaagaagcaacaggagcagcagaagggcgacggtgacaaggtggccaaggagacttctacatccacagagagcttggtcactac
ggacgtcggcgctgtcagcaaccacggcagcagcagcagtgaggtgcctgtcacaagcccacactcgccgacactactcagtcccg
cgtctgacagcgattgtacgcccgtcggcagccctcgagctcgccacggctggggttctgatgctctcagcctgatgctcgacgccgc
cgaattctctactctcttctgatctggtctctggcactctgagcgtgctattcccaggaaatcaaacacacacaa-3’
*Pyricularia grisea* MAT1-2 region (SEQ ID NO:90) 
5’-tctcattttctattcaggtttattaagttattgaggaaccaactggggaccccacttcgtttctttattaactcgaccttcgaacccaggaa
cccaactccctccaagactccgacatcaccaaaaccacaagactctttccattcgattgcgctagaactactcacaaacctcagattgca
aacacaaaccccatcgtcagatcattgaaacgaggcacatctacaatggattcattgcagatggactcctctcaaatggaggcgttgtcg
tcgatttgggtcatcgtgcagcatcagctcgcccaaatcaacaattccaacaacatagctcatatcggcgccgttctcctcaaccaacttg
aggcgcctttcaggagcactttgacatcatggtttcagcagtaagttctccataagctcaaggcttttccgcaagggaaactaacatcagg
taggcacaccggcatcaaccaggtctcagtcgtgcgcgacatcaaggctcgtcgacactacatcggaccaactgatgcttttcaggag
agctatcaagttgtacacatccagggtcacggatttgtctggctttgggatctccaggttgatggtgaggatgccagcatcaattcctgca
atatctctgccgagacagcacagaacttttcccagctgcgtcttgacgagatggcgcctaccaatgatgttacgatggtaggtgatgaga
gcctcatcaacggcaacggcattgactttgccatgttggaagacatcggaagccccgcatcaaacggctcctcattcccgagccctcaa
gttttcacccgggaaatttcattcgatgatctaccagttggcattatgactgccgaggccattcagagagaccttcttggcgccaatattcg
cccggctaacctcattgatccgagtcttgcgagcaatcagaatgtccacaacatcatccatcagaataacattgcccaggcttttacagat
cacctgaacatgatggcatccaatgctgtccacgatgacacagaagagggtctgacagccgcggatcttatgccggcgttgcatcgca
tgttcgtgccgggtcttgagtcaattgcaatggagcagttccacaccacgcttcctaatggcgcactccttcacctgtcaattcctgtcgat
 
gaacctgaggatgctgtctcgaccaccgcgcctgctgctcaacagacttccttgcaagacaagatccctcgtcctgcgaatgcctacat
cctgtaccgcaaggattggcatcccatagtcaagtctgcaaatccaggaatccataacaacgaaatctgtgagttcttttacctccaaactt
ccctagctgctgctaactttttttcctttagcaaaaatcctggggaagcagtgggcagctgagacaccggaagtgcgggctgagtacaa
ggagctcgcggaggagaagaagagagagttctacgcaaagtaccctacatatcgctactctcctcgtcggccgagtgagataatgcg
ccgagtgagtttgtaacacattattgcgtgaaagagtggacagcgagctaacaaattacttgccaacaagaacacgaaaagccgaaag
accccaacgaacggggcagcgaagccgagcaaagcaaagaagtgcggcaacggggttactggtggagcagacagtgcagtcgat
gtcagctcccggggatcgccattttagatgaggtcaccattcacatcatccgagatacattgtacaagtcaggagcagttatcaaagctg
catttcaccagagtcctcatatctgattttttcttctttcttttcacattgccccatcgtacatccagagagctggactcgaaataccctctcaat
agagtctgcaaagaagatcttaggacctacgacataggttacgtggtcattattcaccaagaactacatatctgccagcgaaacatattca
gttttcagagccatttgtcgaaagaaattttgagatgccggccctctggtcaccagtcaaagtttgcggcatcgtttcggagctcaggtaa
gccgacccagagtcctcacaccagagcgacagctcttcagcatctaacgatcacaggctaacacttttggcgcctcaattagcaagaa
catggcctgcatggtttggatctgggtcgtctgcgttcctcagaagcggtaccgcatcacgacagctcgctggaactccagtgggctgc
tggttggccgcgttgattgtgagttttctactttcttcaccttttccttccgtcccagatctgagcagagactgacaagcgacacaaaacctc
cccacagctcctcctcaatctgccaccacagcgtcctcacagtcatcgtcattcgacggcagtgaggaagacacaaacacaaacatca
acgccaatggtatcgatgagagcctgctgccgcctgctctccgcggccgcactggaccttccaagcctccaaaccgctggattctctac
cgtgccgccaagtcagctgagctgcgggcagacaacccatccatgaacgcgagcgaaatctgtaagttcttcgctcttttcactctttttc
tccgctaccacttgtacctaccttttccccatctcccatactctttgaaaagtctccagtcccacctctcaagctgctgttttgacaagataaa
agactaaccaacatctctctcctcacacagctcaagttgcatccctgatgtggcaggcagagtccgccgcgacaaaggcagagtggg
aggagcgcgcagccgaagcccgcgaggagcacatgcgaaacactccagagtatgcggtggcggccggtctgcgccagcgcgcc
atcgccgagaggagaaggcagaggcagcagaagaagaagcaacaggagcagcagaagggcgacggtgacaaggtggccaag
gagacttctacatccacagagagcttggtcactacggacgtcggcgctgtcagcaaccacggcagcagcagcagtgaggtgcctgtc
acaagcccacactcgccgacactactcagtcccgcgtctgacagcgattgtacgcccgtcggcagccctcgagctcgccacggctgg
ggttctgatgctctcagcctgatgctcgacgccgccgaattctctactctcttctgatctggtctctggcactctgagcgtgctattcccagg
aaatcaaacacacacaa-3’
*Pyricularia grisea* spo11 (SEQ ID NO:91)

5'-atgatgctcaggcgctcggggcgtgtccgcaagcctccgaggtggctgctgctgtcctctgttcccgtggaaccccaagatcaag
cgaacagtcaaaatactcgagctagtgagaatcgtcaagagcttgccatgcaagatcaaggttctgatagccgatattctcgggacaga
agccagagcgctgagcggtttgtggaacacggcaatgaatatgtacagcaacaggctatcatccatactgccagccggtcacctagca
atgcagagagccatcctgtgactgtgatctcgcagattgaagccatcttcgagtcgattgcagtcgatatacagcagggcagggaatta
actattccgtattgtcgtgtcagaggcacacgtgacatcaacagccggctaagatttccaggacgcagtgtttctgaggcaaggtcgttt
ggtaagattcctttgcccaccccggggaagcggcaaattacatttctgcgagctgtatttaaattatcatatttctgtagcccgggtactgtg
cattctggagatgtcacaccaggctctcataagtggcaatattgtaacaaaaaggtgtgttaataacatcggatgtagttctttttggctgac
gcgaaaccaacaaggtacgcagagggatggatacacttactaattgcgcaccaggaacatattttaccaagccaaagctctattccgcg

accaagctgttgttgatcgactggtagatgatgtggcattcacactcggagttgggcgacattcattgaacattgtcagtcgcccagagc
agtagcagtagcagtatctgatggcaacggctgacccatctgaagaccgccgctggcaagggtctggtcatagggccaatttcctttgt
cctcaatgatggcacgatggtctcttgcgaccacatgcggactgaggtaatcaccccgataacatactcctgaatggattgcttctggtct
tgtcatcaaattgattggaatacttatccagggaacaattatttgctcaacagaaggactaggtcaaattgagttcccttccgcatgttgggt
cttgattatcgagaaagaagtgagcggaatacatctgcagaaatattggtgctcacaacactgacattcgttttgcaaggcaactttcagg
actcttgcgtcttcccagttcttcaggaactcgcgcgccgggccgggtatcatgatcacagcaagtcaccgccctaaaagtttggctggc
atagcctcaactgagggacggtctggctaatcacaaccttttgatcgtcacagggaaaaggctatccggacctcaatacgaggaacctg
gtgtattgtcttcgagacgcgcgtccgtggatcccgatttacaccttggtcgattatgaccctgacgggatcagaatactgtgcacgtata
aatacggcagctcttctttggagcacgagagacgatcggctgtgaacagcatcgagtggataggaattcgcttttcggacgtcaaacag
cactatctgagaccatgcaacagtctcttgcaggatgatggctcccaaatcaacattgaaggtacgtgccatcacaaagaaagtcaccct
caacaaggaagactttccaagccaagaagctgcggccctccataccaagggctgcgacgctgagaccaggagcctctatctaactca
agtactctccaatatgtagcacactcaagattggacgtagtacaggtcagaagtcggccaggattttctatgccacgacagaacgatttc
gaagcaagcacgtcccagctgacaaccagggaccgtaaacttgcccatgagatggtttcaaggctatctggatcattagatatgaggg
gagaacatgaagagcacttgcgcgagcttcaggtcatgcttatgctcaatgtcaaggccgagatacaagctgttgacaatcttggggat
attaccgagtggattgacaacaaactttgcgcccagctgatgggtgcatag-3’

The disclosure of Japanese Patent Application No. 2022-196304 filed on December 8, 2022 is incorporated herein by reference in its entirety. All literature, patent applications, and technical standards disclosed herein are incorporated herein by reference to the same extent as if they were specifically and individually incorporated by reference.

## Claims

1. A method of preparing a genome-edited cell, the method comprising:
fusing cells; and
introducing into the cells before, during, or after the fusing: a donor nucleic acid; and a protein that promotes at least one selected from the group consisting of cell fusion, nuclear fusion, and meiosis, or a gene thereof,
wherein a nucleic acid contained in the donor nucleic acid is introduced into a genome of the cell during meiosis process of the fused cell.

2. The method of preparing a genome-edited cell according to claim 1, wherein at least one selected from the group consisting of the fusing of the cells, the introducing of the donor nucleic acid, and the introducing of the protein or the gene thereof is performed by bringing the cells into contact with polyethylene glycol, infecting the cells with a virus, or by using an electrical technique.

3. The method of preparing a genome-edited cell according to claim 1, wherein the cells are plant cells, fungal cells, or animal cells.

4. The method of preparing a genome-edited cell according to claim 1, wherein the cells are filamentous fungal cells.

5. The method of preparing a genome-edited cell according to claim 1, wherein the protein or the gene thereof includes at least one selected from the group consisting of a cell fusion gene, a nuclear fusion gene, a mating-related gene, a meiosis-related gene, and a protein encoded by any of these genes.

6. The method of preparing a genome-edited cell according to claim 5, wherein:
the cell fusion gene comprises at least one selected from the group consisting of symc, symb, ham5, ste11, pmk1, whi2, mak1, mak2, and so;
the nuclear fusion gene comprises at least one selected from the group consisting of kar5 and prm1;
the mating-related gene comprises at least one selected from the group consisting of pro 1 and a gene within a MAT idiomorph; and
the meiosis-related gene comprises at least one selected from the group consisting of rad51, ddnm1, CD, CDA, Ung, mre11, rec8, and spo11.

7. The method of preparing a genome-edited cell according to claim 6, wherein the gene within a MAT idiomorph comprises at least one selected from the group consisting of MAT1-2-1 (MAT hmg1), MAT1-2-2 (MAT hmg2), MAT1-1-1 (MAT α-1), MAT1-1-2 (MAT α-2), and MAT1-1-3 (MAT α-3).

8. The method of preparing a genome-edited cell according to claim 1, wherein introduction of the nucleic acid contained in the donor nucleic acid into the genome of the cell occurs by homologous recombination.

9. The method of preparing a genome-edited cell according to claim 1, further comprising introducing a nuclease or a gene thereof into the cells before the meiosis.

10. A method of promoting hybridization, the method comprising
fusing two or more types of cells having different traits; and
introducing a protein that promotes at least one selected from the group consisting of cell fusion, nuclear fusion, and meiosis, or a gene thereof, a nuclease or a gene thereof, or a combination thereof, into the two or more types of cells before, during, or after the fusing,
wherein the two or more types of cells are caused to hybridize during meiosis of the fused cell.

11. The method of promoting hybridization according to claim 10, wherein at least one selected from the group consisting of: the fusing of the cells; and the introducing of the protein or the gene thereof or the nuclease or the gene thereof, is performed by bringing the cells into contact with polyethylene glycol, infecting the cells with a virus, or by using an electrical technique.

12. The method of promoting hybridization according to claim 10, wherein the cells are plant cells, fungal cells, or animal cells.

13. The method of promoting hybridization according to claim 10, wherein the cells are filamentous fungal cells.

14. The method of promoting hybridization according to claim 10, wherein the protein or the gene thereof includes at least one selected from the group consisting of a cell fusion gene, a nuclear fusion gene, a mating-related gene, a meiosis-related gene, and a protein encoded by any of these genes.

15. The method of promoting hybridization according to claim 14, wherein:
the cell fusion gene comprises at least one selected from the group consisting of symc, symb, ham5, ste11, pmk1, whi2, mak1, mak2, and so;
the nuclear fusion gene comprises at least one selected from the group consisting of kar5 and prm1;
the mating-related gene comprises at least one selected from the group consisting of pro 1 and a gene within a MAT idiomorph; and
the meiosis-related gene comprises at least one selected from the group consisting of rad51, ddnm1, CD, CDA, Ung, mre11, rec8, and spo11.

16. The method of promoting hybridization according to claim 15, wherein the gene within a MAT idiomorph comprises at least one selected from the group consisting of hmg1, hmg2, α-1, α-2, and α-3.

17. The method of promoting hybridization according to claim 10, wherein the nuclease or the gene thereof comprises at least one selected from the group consisting of a Cas protein, TALEN, ZFN, a restriction enzyme, and a gene of these.
